# EUROPEAN PATENT SPECIFICATION

(11) **EP 4 297 858 B1**
(45) Date of publication and mention of the grant of the patent: **22.07.2026**
(21) Application number: 22710928.7
(22) Date of filing: 24.02.2022
(51) Int. Cl.: A61N 1/36, A61N 1/372, A61N 1/05

(54) **NEUROSTIMULATION TITRATION SYSTEM**
SYSTEM ZUR NEUROSTIMULATIONSTITRATION
SYSTÈME DE TITRAGE PAR NEUROSTIMULATION

(30) Priority: 24.02.2021 US 202163152861 P
(43) Date of publication of application: 03.01.2024
(73) Proprietor: Medtronic, Inc., Minneapolis, Minnesota 55432 (US)
(72) Inventor: SHARMA, Vinod, Minneapolis, Minnesota 55432 (US); CLELAND, Andrew J., Minneapolis, Minnesota 55432 (US); TORGERSON, Nathan A., Minneapolis, Minnesota 55432 (US)
(74) Representative: Zimmermann & Partner Patentanwälte mbB
(86) International application number: PCT/US2022/070805
(87) International publication number: WO 2022/183194

(56) References cited:
- WO-A1-2010/051403
- WO-A1-2018/080753
- US-A1- 2016 059 007
- US-A1- 2018 154 144
- US-A1- 2019 388 692

## Description

### TECHNICAL FIELD

This disclosure generally relates to medical devices, and more specifically, electrical stimulation.

### BACKGROUND

Electrical stimulation devices, sometimes referred to as neurostimulators or neurostimulation devices, may be external to or implanted within a patient, and configured to deliver electrical stimulation therapy to various tissue sites to treat a variety of symptoms or conditions such as chronic pain, tremor, Parkinson's disease, epilepsy, or other neurological disorders, urinary or fecal incontinence, sexual dysfunction, obesity, or gastroparesis. An electrical stimulation device may deliver electrical stimulation therapy via electrodes, e.g., carried by one or more leads, positioned proximate to target locations associated with the brain, the spinal cord, pelvic nerves, tibial nerves, peripheral nerves, the gastrointestinal tract, or elsewhere within a patient. Stimulation proximate the spinal cord, proximate the sacral nerve, within the brain, and proximate peripheral nerves is often referred to as spinal cord stimulation (SCS), sacral neuromodulation (SNM), deep brain stimulation (DBS), and peripheral nerve stimulation (PNS), respectively.

A physician or clinician may select values for a number of programmable stimulation parameters in order to define the electrical stimulation therapy to be delivered by the implantable stimulator to a patient. For example, the physician or clinician may select one or more electrodes, polarities of selected electrodes, a voltage or current amplitude, a pulse width, and a pulse frequency as stimulation parameters. A set of therapy stimulation parameters, such as a set including electrode combination, electrode polarity, amplitude, pulse width and pulse frequency, may be referred to as a therapy program in the sense that they define the electrical stimulation therapy to be delivered to the patient. Documents WO 2010/051403 A1, WO 2018/080753 A1 and US 2018/154144 A1 relate to stimulation devices.

### SUMMARY

The invention is defined by the independent claims. In general, the disclosure describes techniques for titrating neurostimulation from a loading dose to a lesser maintenance dose based on patient feedback in response to the loading dose, where delivering the maintenance dose consumes less power than delivering the loading dose. In some examples, a first device delivers the loading dose and a second device delivers the maintenance dose. In some examples, the second device is not capable of delivering the loading dose independently, e.g., the second device may be powered by a lower-powered rechargeable or primary cell.

**One** or more neurostimulation devices, external programmers, or remote programming device may receive patient feedback from one or more sensing and/or patient-input devices, either directly or via network connections, and perform, direct or control, based on the patient feedback, automatic control of neurostimulation dosing. The one or more neurostimulation devices, external programmer, or remote programming device may determine, based on the patient feedback, a maintenance dose that consumes less power than an initial loading dose delivered over a first period of time. The neurostimulation device may deliver one or more maintenance doses to the patient over a second period of time.

In one example, this disclosure describes a method of titrating a therapy including determining, for a patient, a loading dose of electric stimulation; delivering, via a device and during a first time period, the loading dose to the patient; receiving patient feedback representing a response of the patient to the loading dose; determining, based on patient feedback, a maintenance dose of electrical stimulation; and delivering, via the device and during a second time period that is after the first time period, a maintenance dose of electrical stimulation, wherein delivering the maintenance dose of electrical stimulation consumes less power than delivering the loading dose of electrical stimulation.

In another example, this disclosure describes a system that includes electrodes configured to deliver the electrical stimulation to a patient; and a device comprising processing circuitry configured to: determine, for a patient, a loading dose of electric stimulation; cause electrical stimulation circuitry to deliver, during a first time period, the loading dose to the patient; receive patient feedback representing a response of the patient to the loading dose; determine, based on patient feedback, a maintenance dose of electrical stimulation; and cause the electrical stimulation circuitry to deliver, and during a second time period that is after the first time period, a maintenance dose of electrical stimulation, wherein delivering the maintenance dose of electrical stimulation consumes less power than delivering the loading dose of electrical stimulation.

In another example, this disclosure describes a computer readable medium comprising instructions that when executed cause one or more processors to: determine, for a patient, a loading dose of electric stimulation; cause a first stimulator device to deliver the loading dose to the patient during a first time period; receive patient feedback representing a response of the patient to the loading dose; determine, based on patient feedback, a maintenance dose of electrical stimulation; and cause a second stimulator device that is implantable in the patient to deliver the maintenance dose to the patient during a second time period, wherein delivering the maintenance dose of electrical stimulation consumes less power than delivering the loading dose of electrical stimulation, wherein the second stimulator device is not independently capable of delivering the loading dose, wherein the second stimulator device is capable of delivering a rescue does when the second stimulator device is connected to an external power source, wherein the first stimulator device is configured to deliver the loading dose via at least one electrode included with at least one lead, wherein the second stimulator device is configured to deliver the maintenance dose via the at least one electrode included with the at least one lead.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 is a conceptual diagram illustrating an example system that includes an implantable medical device (IMD) in the form of a neurostimulation device configured to deliver spinal cord stimulation (SCS), an external programmer, and one or more sensing devices in accordance with one or more techniques of this disclosure.
FIG. 2A is a block diagram illustrating an example of an IMD in the form of a neurostimulation device, in accordance with one or more techniques of this disclosure.
FIG. 2B is a block diagram illustrating an example of an IMD in the form of a neurostimulation device, in accordance with one or more techniques of this disclosure.
FIG. 3 is a block diagram illustrating an example of an external programmer suitable for use with the IMD of FIG. 2, in accordance with one or more techniques of this disclosure.
FIG. 4 is a flow diagram illustrating an example method of titrating a therapy, in accordance with one or more techniques of this disclosure.
FIG. 5 is a plot of an example of titrating electric stimulation doses, in accordance with one or more techniques of this disclosure.
FIG. 6 is a plot of another example of titrating electric stimulation doses, in accordance with one or more techniques of this disclosure.
FIG. 7 is a plot of another example of titrating electric stimulation doses, in accordance with one or more techniques of this disclosure.
FIG. 8 is a plot of another example of titrating electric stimulation doses, in accordance with one or more techniques of this disclosure.
FIG. 9 is a plot of another example of titrating electric stimulation doses, in accordance with one or more techniques of this disclosure.

### DETAILED DESCRIPTION

Stimulation therapy (e.g., including spinal cord stimulation, tibial nerve stimulation, etc.) may provide pain relief and/or other therapeutic benefits. In some circumstances, constant delivery of electrical stimulation doses may be required to achieve the desired pain relief and/or other therapeutic benefits. In other circumstances, electrical stimulation may have a durable effect such that constant delivery of electrical stimulation is not required to achieve the desired pain relief and/or other therapeutic benefits. Where electrical stimulation has such a durable effect, a device may deliver electrical stimulation to a patient in accordance with a treatment program that proscribes on-periods in which the device delivers electrical stimulation doses of the treatment program and off-periods in which the device does not deliver electrical stimulation doses of the treatment program.

In accordance with one or more techniques of this disclosure, an electrical stimulation system may titrate the dosing of the stimulation therapy based on patient feedback. For instance, the electrical stimulation system may titrate the dosing such that a loading dose is delivered for a first period of time, a maintenance dose that is less than the loading dose may be determined based on patient feedback in response to the loading dose, and the maintenance dose may be delivered thereafter for a second time period. In some examples, a loading dose may be an amount of electric stimulation therapy that may be delivered to provide at least a threshold level of pain relief and/or other therapeutic benefits and which may initiate the durable effect of the stimulation therapy which may thereafter be maintained via delivery of "smaller" maintenance doses, e.g., maintenance doses that are an amount of electric stimulation therapy that is less than the loading dose. A maintenance dose may be an amount of electric stimulation therapy that is less than the loading dose and that may be delivered to refresh, prolong, extend, continue, etc., the threshold level of pain relief and/or other therapeutic benefits. In other words, a maintenance dose may be a dose smaller than the initial loading dose capable of prolonging the durable effect and/or maintaining the threshold level of pain relief and/or benefits for at least a second period of time after the threshold level has been initiated via the loading dose. By reducing the dosing from the loading dose to the maintenance dose, the system may conserve battery life and/or reduce the development of a tolerance to the stimulation therapy by the patient.

The system may determine further dosing after the second time period based on further received patient feedback. For example, if the patient is doing well, the system may continue to deliver the maintenance dose or may further reduce the dosing (e.g., further titrate), based on the patient feedback, to a second maintenance dosing. If a patient started to do worse, the system may deliver a rescue dose during a third period of time. In some examples, the system may deliver a rescue dose that is greater than the maintenance dose and, in some examples, may be the loading dose. By reducing the dosing from the loading dose to the maintenance dose while also providing the option to deliver a rescue dose (which may be substantially similar to the loading dose) during a third time period, the system may conserve battery life and/or reduce the development of a tolerance to the stimulation therapy while also providing flexibility to tailor electric stimulation to the patient's needs at a particular period in time.

In some examples, the system may deliver the loading dose via a first device and the maintenance dose via a second device. For example, the second device may be smaller, lighter weight, and consume less power than the first device. In some examples, the second device may not be independently capable of delivering an amount of dosing that is more than the maintenance dosing, e.g., a rescue and/or loading dose, but may be configured to deliver an amount of dosing that is more than the maintenance dose, e.g., the rescue and/or loading dose with assistance, such as when connected to an external power source. By delivering the maintenance dose with a second device that consumes less power than the first device and may be smaller and lighter, the system may reduce the size of device to be implanted in the patient and utilize a smaller, lighter, and less costly power source such as a rechargeable and/or primary cell that is lower-powered than the power source of the first device.

The system may receive patient feedback including subjective feedback and/or objective feedback. The system may receive subjective patient feedback including a binary pain response (e.g., press a button when there is pain), a pain score, an area of pain, an amount of paresthesia, or an area of paresthesia pain scores, voids per day, and the like. The system may receive objective patient feedback including biomarkers, physiological signals, or electronically sensed outcomes such as an evoked compound action potential (ECAP), a local field potential (LFP), a heart rate, a heart rate variability, a blood flow, or a galvanic skin response, or a patient posture or behavior, such as a patient position, a patient movement, a patient movement history over a predetermined amount of time, a history of patent-selected stimulation parameters over a predetermined amount of time.

The power consumed and/or required to deliver the loading dose and the maintenance dose may be a consequence of the stimulation parameters, namely, a combination of selected electrodes, the polarities of the selected electrodes, the voltage or current amplitude, pulse width, and pulse frequency delivered by each electrode, and the stimulation cycling, e.g., the rate and length of time (e.g., on-time) of each dose. The amount of stimulation, and the amount of power consumed by the device delivering the stimulation, may be reduced by reducing any or all of the amplitude, pulse width, pulse frequency, cycling rate, and cycling on-time.

In some examples, the system may alter settings or the occurrence of the applied therapy so that the therapy is not provided more than necessary, e.g., to save battery life, but also to provide the therapy frequently enough so that the outcome is consistent and beneficial when the therapy is not being provided.

In some examples, e.g., for fully implantable systems, a patient may use a user interface on a patient's phone or on their patient programmer to input feedback, such as pain scores. The system may titrate electric stimulation by reducing the amount of electric stimulation of each dose (e.g., the electrode combination, amplitude, pulse width, and pulse frequency) and also the cycling of the electric stimulation doses, e.g., the cycle frequency, on-time, off-time, and/or duty cycle of the delivery of the doses, to balance the therapy between therapeutic effects, potential development of tolerance, and device battery life based on the feedback and/or feedback trends.

In some examples, the system may enable the use of a primary cell therapy delivery device instead of a rechargeable therapy delivery device by cycling and/or tapering the titration of the therapy, for example, for therapies that have been using higher dose settings that may use more electrical energy. In some examples, tapering titration of the electric stimulation from a loading dose to a maintenance dose may be used with durable-effect therapies with long (days) or short (minutes) durable effect timescales. In some examples, the system may taper titration of the electric stimulation therapy from a loading dose to a maintenance dose gradually over a period of time, and in some examples the system may titrate the electric stimulation therapy from a loading dose to a maintenance dose immediately after the first time period, e.g., a "step change."

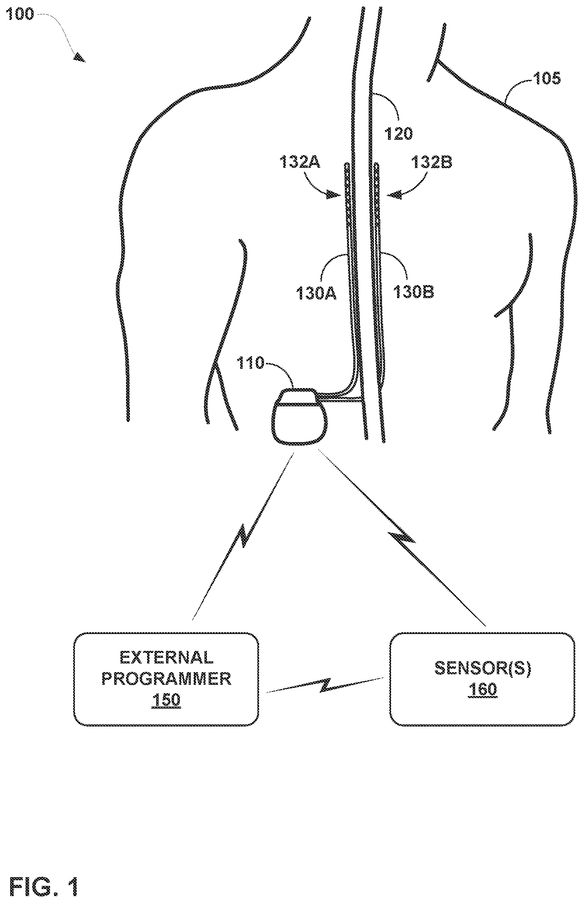

Systems and methods for titrating a neurostimulation therapy based on patient feedback are described herein. The system may include a stimulator system that interacts with a stimulator programmer. FIG. 1 is a conceptual diagram illustrating an example system 100 that includes an implantable medical device (IMD) 110 configured to deliver spinal cord stimulation (SCS) therapy, processing circuitry 140, an external programmer 150, and one or more sensors 160, in accordance with one or more examples of this disclosure. Processing circuitry 140 may include one or more processors configured to perform various operations of IMD 110. Although the examples described in this disclosure are generally applicable to a variety of medical devices including external devices and IMDs, application of such techniques to IMDs and, more particularly, implantable electrical stimulators (e.g., neurostimulators) will be described for purposes of illustration. More particularly, the disclosure will refer to an implantable SCS system for purposes of illustration, but without limitation as to other types of neurostimulation devices or other therapeutic applications of neurostimulation, including an external neurostimulator. For example, the system may not be a fully implanted system where the pulse generator is external to the patient and stimulation is transmitted transdermally. In one or more examples, the stimulators may be configured to deliver peripheral nerve stimulation or spinal nerve root stimulation.

As shown in FIG. 1, system 100 includes an IMD 110, leads 130A and 130B, and external programmer 150 shown in conjunction with a patient 105, who is ordinarily a human patient. In the example of FIG. 1, IMD 110 is an implantable electrical stimulator that is configured to generate and deliver electrical stimulation therapy to patient 105, e.g., for relief of chronic pain or other symptoms, via one or more electrodes 132A, 132B of leads 130A and/or 130B, respectively. In the example of FIG. 1, each lead 130A, 130B includes eight electrodes 132A, 132B respectively, although the leads may each have a different number of electrodes. Leads 130A, 130B may be referred to collectively as "leads 130" and electrodes 132A, 132B may be referred to collectively as "electrodes 132." In other examples, IMD 110 may be coupled to a single lead carrying multiple electrodes or more than two leads each carrying multiple electrodes.

IMD 110 may be a chronic electrical stimulator that remains implanted within patient 105 for weeks, months, or years. In other examples, IMD 110 may be a temporary, or trial, stimulator used to screen or evaluate the efficacy of electrical stimulation for chronic therapy. In one example, IMD 110 is implanted within patient 105, while in another example, IMD 110 is an external device coupled to one or more leads percutaneously implanted within the patient. In some examples, IMD 110 uses electrodes on one or more leads, while in other examples, IMD 110 may use one or more electrodes on a lead or leads and one of more electrodes on a housing of the IMD. In further examples, IMD 110 may be leadless and instead use only electrodes carried on a housing of the IMD.

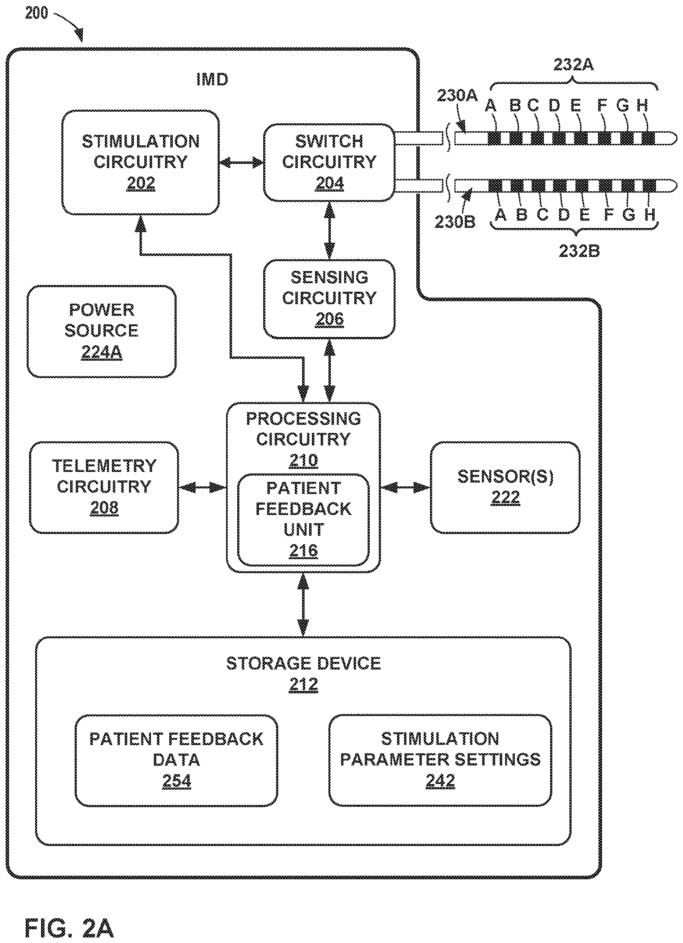

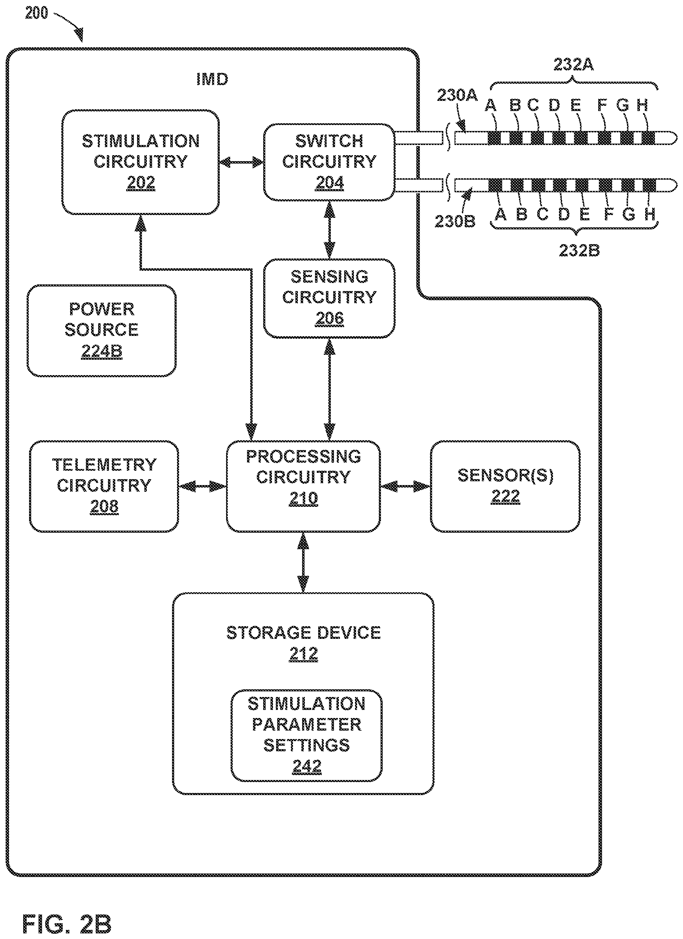

IMD 110 may be constructed of any polymer, metal, or composite material sufficient to house the components of IMD 110 (e.g., components illustrated in FIG. 2A, 2B) within patient 105. In this example, IMD 110 may be constructed with a biocompatible housing, such as titanium or stainless steel, or a polymeric material such as silicone, polyurethane, or a liquid crystal polymer, and surgically implanted at a site in patient 105 near the pelvis, abdomen, or buttocks. In other examples, IMD 110 may be implanted at other suitable sites within patient 105, which may depend, for example, on the target site within patient 105 for the delivery of electrical stimulation therapy. The outer housing of IMD 110 may be configured to provide a hermetic seal for components, such as a rechargeable or non-rechargeable power source. In addition, in some examples, the outer housing of IMD 110 is selected from a material that facilitates receiving energy to charge the rechargeable power source.

In the example of FIG. 1, electrical stimulation energy, which may be delivered as regulated current or regulated voltage-based pulses, is delivered from IMD 110 to one or more target tissue sites of patient 105 via leads 130 and electrodes 132. Leads 130 position electrodes 132 adjacent to target tissue of spinal cord 120. One or more of the electrodes 132 may be disposed at a distal tip of a lead 130 and/or at other positions at intermediate points along the lead. Leads 130 may be implanted and coupled to IMD 110. The electrodes 132 may transfer electrical stimulation generated by an electrical stimulation generator in IMD 110 to tissue of patient 105. Although leads 130 may each be a single lead, a lead 130 may include a lead extension or other segments that may aid in implantation or positioning of lead 130.

The electrodes 132 of leads 130 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes) or any other type of electrodes capable of forming unipolar, bipolar or multipolar electrode combinations for therapy. Ring electrodes arranged at different axial positions at the distal ends of lead 130 will be described for purposes of illustration. Deployment of electrodes via leads 130 is described for purposes of illustration, but electrodes may be arranged on a housing of IMD 110, e.g., in rows and/or columns (or other arrays or patterns), as surface electrodes, ring electrodes, or protrusions.

Neurostimulation stimulation parameters defining the electrical stimulation pulses delivered by IMD 110 through electrodes 132 of leads 130 may include information identifying which electrodes have been selected for delivery of the stimulation pulses according to a stimulation program and the polarities of the selected electrodes (the electrode combination), and voltage or current amplitude, pulse rate (i.e., frequency), and pulse width of the stimulation pulses. The neurostimulation stimulation parameters may further include a cycling parameter that specifies when, or how long, stimulation is turned on and off. Neurostimulation stimulation parameters may be programmed prior to delivery of the neurostimulation pulses, manually adjusted based on user input, or automatically controlled during delivery of the neurostimulation pulses, e.g., based on sensed conditions.

Although the example of FIG. 1 is directed to SCS therapy, e.g., to treat pain, in other examples, system 100 may be configured to treat other conditions that may benefit from neurostimulation therapy. For example, system 100 may be used to treat tremor, Parkinson's disease, epilepsy, or other neurological disorders, urinary or fecal incontinence, sexual dysfunction, obesity, or gastroparesis, or psychiatric disorders such as depression, mania, obsessive compulsive disorder, or anxiety disorders. Hence, in some examples, system 100 may be configured to deliver sacral neuromodulation (SNM), deep brain stimulation (DBS), peripheral nerve stimulation (PNS), or other stimulation, such as peripheral nerve field stimulation (PNFS), cortical stimulation (CS), gastrointestinal stimulation, or any other stimulation therapy capable of treating a condition of patient 105. In some examples, system 100 may be configured where the electrical stimulation includes stimulation parameters to deliver therapy to address a condition of one or more of painful diabetic neuropathy (PDN), peripheral vascular disease (PVD), peripheral artery disease (PAD), complex regional pain syndrome (CRPS), angina pectoris (AP), leg pain, back pain or pelvic pain.

Leads 130 may include, in some examples, one or more sensors configured to sense one or more physiological stimulation parameters of patient 105, such as patient activity, pressure, temperature, posture, heart rate, blood flow, or other characteristics. At least some of electrodes 132 may be used to sense electrical signals within patient 105, additionally or alternatively to delivering stimulation. IMD 110 is configured to deliver electrical stimulation therapy to patient 105 via selected combinations of electrodes carried by one or both of leads 130, alone or in combination with an electrode carried by or defined by an outer housing of IMD 110. The target tissue for the electrical stimulation therapy may be any tissue affected by electrical stimulation. In some examples, the target tissue includes nerves, smooth muscle or skeletal muscle. In the example illustrated by FIG. 1, the target tissue is tissue proximate spinal cord 120, such as within an intrathecal space or epidural space of spinal cord 120, or, in some examples, adjacent nerves that branch off spinal cord 120. Leads 130 may be introduced into spinal cord 120 in via any suitable region, such as the thoracic, cervical or lumbar regions.

Stimulation of spinal cord 120 may, for example, prevent pain signals from being generated and/or traveling through spinal cord 120 and to the brain of patient 105. Patient 105 may perceive the interruption of pain signals as a reduction in pain and, therefore, efficacious therapy results. In some examples, stimulation of spinal cord 120 may produce paresthesia which may reduce the perception of pain by patient 105, and thus, provide efficacious therapy results. In other examples, stimulation of spinal cord 120 may be effective in reducing pain with or without presenting paresthesia. In some examples, some electrical stimulation pulses may be directed to glial cells while other electrical stimulation (e.g., delivered by a different electrode combination and/or with different stimulation parameters) is directed to neurons. In other examples, stimulation of spinal cord 120 may be effective in promoting blood flow in one or more remote tissue locations, e.g., in a limb or appendage, thereby alleviating or reducing pain or other symptoms, or preventing or delaying onset of tissue damage or degeneration.

IMD 110 generates and delivers electrical stimulation therapy to a target stimulation site within patient 105 via the electrodes of leads 130 to patient 105 according to one or more therapy stimulation programs. A therapy stimulation program specifies values for one or more stimulation parameters that define an aspect of the therapy delivered by IMD 110 according to that program. For example, a stimulation therapy program that controls delivery of stimulation by IMD 110 in the form of stimulation pulses may define values for voltage or current pulse amplitude, pulse width, and pulse rate (e.g., pulse frequency) for stimulation pulses delivered by IMD 110 according to that program, as well as the particular electrodes and electrode polarities forming an electrode combination used to deliver the stimulation pulses. Hence, a stimulation therapy program may specify the location(s) at which stimulation is delivered and amplitude, pulse width and pulse rate of the stimulation. In some examples, a stimulation therapy program may specify cycling of the stimulation, e.g., in terms of that when, or how long, stimulation is turned on and off.

A user, such as a clinician or patient 105, may interact with a user interface of an external programmer 150 to program IMD 110. Programming of IMD 110 may refer generally to the generation and transfer of commands, programs, or other information to control the operation of IMD 110. In this manner, IMD 110 may receive the transferred commands and programs from external programmer 150 to control electrical stimulation therapy. For example, external programmer 150 may transmit therapy stimulation programs, stimulation parameter adjustments, therapy stimulation program selections, user input, or other information to control the operation of IMD 110, e.g., by wireless telemetry or wired connection.

In some cases, external programmer 150 may be characterized as a physician or clinician programmer if it is primarily intended for use by a physician or clinician. In other cases, external programmer 150 may be characterized as a patient programmer if it is primarily intended for use by a patient. A patient programmer may be generally accessible to patient 105 and, in many cases, may be a portable device that may accompany patient 105 throughout the patient's daily routine, e.g., as a handheld computer similar to a tablet or smartphone. For example, a patient programmer may receive input from patient 105 when the patient wishes to terminate or change stimulation therapy. In general, a physician or clinician programmer may support selection and generation of programs by a clinician for use by IMD 110, and may take the form, for example, of a handheld computer (e.g., a tablet computer), laptop computer or desktop computer, whereas a patient programmer may support adjustment and selection of such programs by a patient during ordinary use. In other examples, external programmer 150 may include, or be part of, an external charging device that recharges a power source of IMD 110. In this manner, a user may program and charge IMD 110 using one device, or multiple devices.

IMD 110 and external programmer 150 may exchange information and may communicate via wireless communication using any techniques known in the art. Examples of communication techniques may include, for example, radiofrequency (RF) telemetry and inductive coupling, but other techniques are also contemplated. In some examples, external programmer 150 includes a communication head that may be placed proximate to the patient's body near the IMD 110 implant site to improve the quality or security of communication between IMD 110 and external programmer 150. Communication between external programmer 150 and IMD 110 may occur during power transmission or separate from power transmission.

IMD 110, in response to commands from external programmer 150, may deliver electrical stimulation therapy according to a plurality of therapy stimulation programs to a target tissue site of the spinal cord 120 of patient 105 via electrodes 132 on leads 130. In some examples, IMD 110 automatically modifies therapy stimulation programs as therapy needs of patient 105 evolve over time. For example, the modification of the therapy stimulation programs may cause the adjustment of at least one parameter of the plurality of stimulation pulses based on received information.

IMD 110 and/or external programmer 150 may receive information from one or more sensors 160, e.g., directly via wireless communication or indirectly from an intermediate server via a network connection. Sensor 160 may be positioned to sense one or more physiological responses at a selected location on patient 105. In some examples, sensor 160 may be positioned at, attached to or near tissue for a target anatomical area, e.g., at a limb or appendage, such as at or on a leg, toe, foot, arm, finger or hand of patient 105, e.g., to sense a galvanic skin response adjacent to placement of sensor 160. In some examples, sensor 160 may be attached to an appendage of the patient 105 to sense a physiological response associated with the appendage, e.g., by a clip-on mechanism, strap, elastic band and/or adhesive. In some examples, sensor 160 (or one of a plurality of sensors 160) may be implantable within patient 105, e.g., within a limb or appendage of the patient, near the spinal cord of the patient, within the brain of the patient, and the like.

In some examples, sensor 160 may be a physiological and/or patient posture or behavior sensor. For example, sensor 160 may be a heart rate monitor configured to detect and/or determine a heart rate and/or a heart rate variability. Sensor 160 may be configured to detect and/or determine a galvanic skin response, or to detect and/or determine a biopotential. Sensor 160 may be a thermometer configured to detect and/or determine a temperature of at least a part of the patient's anatomy. Sensor 160 may be configured to measure a pressure, e.g., a patient blood pressure, or to measure an impedance of at least a portion of the patient's anatomy. Sensor 160 may be a blood flow sensor that measures blood flow and provides information related to blood flow associated with tissue of the patient. For example, sensor 160 may provide blood flow values, or other information indicative of blood flow values or changes in blood flow values. The blood flow value may be an instantaneous blood flow measurement or may be a measurement of blood flow over a period of time such as average blood flow value, maximum blood flow value, minimum blood flow value during the period of time. In some examples, sensor 160 may be a microphone configured to detect/determine sounds of at least a portion of the patient's anatomy. In some examples, sensor 160 may at least partially comprise electrodes 132A, 132B. For example, sensor 160 may be configured to detect and/or determine ECAPs, LFPs, a network excitability, and the like. In some examples, sensor 160 may comprise and accelerometer configured to detect and/or determine a position and/or patient movement, a patient movement history over a predetermined amount of time, and the like. In some examples, sensor 160 may be a patient-input device, e.g., external programmer 150, a smartphone or computing device, or any other suitable device, configured to receive and communicate subjective patient feedback. For example, sensor 160 may be configured to receive a pain response, a pain score, an area of pain, an amount of paresthesia, an area of paresthesia, information relating to voiding and/or a voiding rate (e.g., voids per day), and the like. In some examples, sensor 160 may be an environmental sensor, such as a microphone, thermometer, hygrometer, pressure sensor, and the like, configured to detect and/or determine sounds, temperatures, humidity and pressure, etc., of the environment in which the patient is located.

In accordance with one or more aspects of this disclosure, system 100 and/or IMD 110 and/or external programmer 150 may be configured to titrate and/or taper a titration of electric stimulation therapy, e.g., from a loading dose to a lesser maintenance dose. For example, a physician or clinician, IMD 110, or external programmer 150 may be configured to determine a loading dosing of electric stimulation. A "dose" of electric stimulation may comprise delivery of electric stimulation pulses according to one or more specified electric stimulation parameters, e.g., an electrode combination, an amplitude, a pulse frequency, and a pulse width, as well as delivery of one or more pulses according to a cycling of the pulses. In some examples, system 100 and/or IMD 110 and/or external programmer 150 may titrate and/or change a dose and/or electric stimulation dosing by titrating the electric stimulation parameters, the cycling, or both. For example, IMD 110 may titrate electric stimulation therapy from a loading dose to a maintenance dose by reducing the electric stimulation pulses, a cycle frequency of delivery of the pulses, e.g., the rate at which electric stimulation pulses are delivered, reducing the on-time of the individual pulses, increasing to off-time between individual pulses, and/or reducing the duty cycle of the cycling, e.g., the ratio of stimulation pulse delivery on-time to off-time. The cycle frequency may be specified in a variety of manners, such as time between dose delivery (e.g., in seconds, minutes, hours, days, weeks, months, etc.), an amount of time for both on-time and off-time (e.g., on-time/off-time pairs in second, minutes, hours, days, weeks, months, etc.), as a schedule specifying both an on-time and an off-time for each dose which may be the same or which may vary for each dose, a continuous taper in which the off-time is increased at a constant or variable rate and/or an on-time is decreased at a constant or a variable rate, and the like.

IMD 110 may be configured to deliver a loading dose of electric stimulation, and IMD 110 and/or external programmer 150 may be configured to receive patient feedback representing a response of the loading dose, e.g., via sensor 160. IMD 110 and/or external programmer 150 may be configured to determine a maintenance dose based on the received feedback, and IMD 110 may be configured to deliver the maintenance dose. In this way, IMD 110 may be configured to consume and/or use less electrical power when delivering the maintenance dose relative to delivering the loading dose, and to have an increased battery life. Additionally or alternatively, IMD 110 may be configured to reduce eliminate, reduce, alleviate, or delay stimulation tolerance when delivering the maintenance dose relative to delivering the loading dose.

In some examples, system 100 may be configured to switch devices between delivery of a loading dose and delivery of a maintenance dose. For example, a first IMD 110 may be configured to deliver the loading dose and a second IMD 110 may be configured to deliver the maintenance dose. For example, the first IMD 110 and/or external programmer 150 may be configured to receive patient feedback representing a response of the patient to the loading dose, e.g., via sensor 160. The first IMD 110 and/or external programmer 150 may be configured to determine a maintenance dose based on the received feedback, and the second IMD 110 may be configured to deliver the maintenance dose. In this way, system 100 and/or the second IMD 110 may be configured to deliver the maintenance dose via a smaller, lighter weight, and less expensive IMD, and may use a primary cell IMD or a rechargeable IMD with a smaller rechargeable power source than the first IMD.

FIG. 2A and 2B are block diagrams illustrating example configurations of components of an IMD 200A and an IMD 200B, respectively, in accordance with one or more techniques of this disclosure. IMD 200A and/or IMD 200B may be an example of IMD 110 of FIG. 1, e.g., a first IMD 110 capable of independently delivering a loading dose of electric stimulation and/or a second IMD 110 capable of independently delivering a maintenance dose of electric stimulation but not capable of delivering a loading dose of electric stimulation. In the examples shown in FIGS. 2A and 2B, IMD 200A and IMD 200B each include stimulation generation circuitry 202, switch circuitry 204, sensing circuitry 206, telemetry circuitry 208, sensor(s) 222, lead 230A carrying electrodes 232A, which may correspond to lead 130A and electrodes 132A of FIG. 1, and lead 230B carrying electrodes 232B, which may correspond to lead 130B and electrodes 132B of FIG. 1. In the examples shown in FIG. 2A, IMD 200A includes processing circuitry 210A, power source 224A, and storage device 212A, and in the example shown in FIG. 2B, IMD 200B includes processing circuitry 210B, power source 224B, and storage device 212B. Processing circuitry 210A and/or 210B may include one or more processors configured to perform various operations of IMD 200A and/or IMD 200B. In some examples, IMD 200A may include power source 224B and IMD 200B may include power source 224A.

In the examples shown in FIGS. 2A and 2B, storage devices 212A and 212B store stimulation parameter settings 242. In addition, as shown in FIG. 2A, storage device 212A may store patient feedback data 254 obtained directly or indirectly from one or more sensors 160 (FIG. 1) or from patient via a patient-input device. In this case, IMD 200A of FIG. 2A may process patient feedback information and select or adjust stimulation parameter settings, including cycling, based on the patient feedback information.

In one or more examples, such as shown in FIG. 2B, the IMD 200B may not store or receive the patient feedback information. Instead, external programmer 150 or another device may directly or indirectly select or adjust stimulation parameter settings based on patient feedback information and communicate the selected settings or adjustments to IMD 200B of FIG. 2B. In some examples, stimulation parameter settings 242 may include stimulation parameters (sometimes referred to as "sets of therapy stimulation parameters") for respective different stimulation programs selectable by the clinician or patient for therapy. In some examples, stimulation parameter settings 242 may include one or more recommended parameter settings. In this manner, each stored therapy stimulation program, or set of stimulation parameters, of stimulation parameter settings 242 defines values for a set of electrical stimulation parameters (e.g., a stimulation parameter set), such as electrode combination (selected electrodes and polarities), stimulation current or voltage amplitude, stimulation pulse width, pulse rate, or duty cycle. In some examples, stimulation parameter settings 242 may further include cycling information indicating when or how long stimulation is turned on and off, e.g., periodically and/or according to a schedule. For example, recommended parameter settings may indicate the stimulation to turn on for a certain period of time, and/or to turn off stimulation for a certain period of time. In another example, recommended cycle parameter settings may indicate for stimulation to turn on for a period of time without creating desensitization of the stimulation. In one or more examples, the recommended parameter settings may indicate stimulation to occur at a certain time of day, for example when the patient is typically awake or active, or sleeping. In one or more examples, recommended parameter settings relate to when the patient has a certain posture, for example only deliver stimulation when the patient is in a supine position.

Stimulation generation circuitry 202 includes electrical stimulation circuitry configured to generate electrical stimulation and generates electrical stimulation pulses selected to alleviate symptoms of one or more diseases, disorders or syndromes. While stimulation pulses are described, stimulation signals may take other forms, such as continuous-time signals (e.g., sine waves) or the like. The electrical stimulation circuitry may reside in an implantable housing, for example of the IMD. Each of leads 230A, 230B may include any number of electrodes 232A, 232B. The electrodes are configured to deliver the electrical stimulation to the patient. In the example of FIGS. 2A and 2B, each set of electrodes 232A, 232B includes eight electrodes A-H. In some examples, the electrodes are arranged in bipolar combinations. A bipolar electrode combination may use electrodes carried by the same lead 230A, 230B or different leads. For example, an electrode A of electrodes 232A may be a cathode and an electrode B of electrodes 232A may be an anode, forming a bipolar combination. Switch circuitry 204 may include one or more switch arrays, one or more multiplexers, one or more switches (e.g., a switch matrix or other collection of switches), or other electrical circuitry configured to direct stimulation signals from stimulation generation circuitry 202 to one or more of electrodes 232A, 232B, or directed sensed signals from one or more of electrodes 232A, 232B to sensing circuitry 206. In some examples, each of the electrodes 232A, 232B may be associated with respective regulated current source and sink circuitry to selectively and independently configure the electrode to be a regulated cathode or anode. Stimulation generation circuitry 202 and/or sensing circuitry 206 also may include sensing circuitry to direct electrical signals sensed at one or more of electrodes 232A, 232B.

Sensing circuitry 206 may be configured to monitor signals from any combination of electrodes 232A, 232B. In some examples, sensing circuitry 206 includes one or more amplifiers, filters, and analog-to-digital converters. Sensing circuitry 206 may be used to sense physiological signals, such as ECAP signals and/or LFP signals. In some examples, sensing circuitry 206 detects ECAP and/or LFP signals from a particular combination of electrodes 232A, 232B. In some cases, the particular combination of electrodes for sensing ECAP and/or LFP signals includes different electrodes than a set of electrodes 232A, 232B used to deliver stimulation pulses. Alternatively, in other cases, the particular combination of electrodes used for sensing ECAP and/or LFP signals includes at least one of the same electrodes as a set of electrodes used to deliver stimulation pulses to patient 105. Sensing circuitry 206 may provide signals to an analog-to-digital converter, for conversion into a digital signal for processing, analysis, storage, or output by processing circuitry 210.

Telemetry circuitry 208 supports wireless communication between IMD 200A and/or IMD 200B and an external programmer or another computing device under the control of processing circuitry 210. Processing circuitry 210A and/or 210B of IMD 200A and/or IMD 200B, respectively, may receive, as updates to programs, values for various stimulation parameters such as amplitude and electrode combination, from the external programmer via telemetry circuitry 208. Processing circuitry 210A and/or 210B of IMD 200 A and/or IMD 200B, respectively, may store updates to the stimulation parameter settings 242 or any other data in storage device 212. Telemetry circuitry 208 in IMD 200A and/or IMD 200B, as well as telemetry circuits in other devices and systems described herein, such as the external programmer and patient feedback sensing system, may accomplish communication by radiofrequency (RF) communication techniques. In addition, telemetry circuitry 208 may communicate with an external medical device programmer via proximal inductive interaction of IMD 200A and/or IMD 200B with the external programmer, where the external programmer may be one example of external programmer 150 of FIG. 1. Accordingly, telemetry circuitry 208 may send information to the external programmer on a continuous basis, at periodic intervals, or upon request from IMD 110 or the external programmer.

Processing circuitry 210A and/or 210B may include one or more processors, such as any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210A and/or 210B herein may be embodied as firmware, hardware, software or any combination thereof. Processing circuitry 210A and/or 210B controls stimulation generation circuitry 202 to generate stimulation signals according to stimulation parameter settings 242. In some examples, processing circuitry 210A and/or 210B may execute other instructions stored in storage device 212A and/or 212B, respectively, to apply stimulation parameters specified by one or more of programs, such as amplitude, pulse width, pulse rate, and pulse shape of each of the stimulation signals.

In the illustrated example of FIG. 2A, processing circuitry 210A includes a patient feedback unit 216 to process the patient feedback information. Patient feedback unit 216 may represent an example of a portion of processing circuitry configured to process patient feedback information received from a patient feedback sensor, such as sensor 160, and/or a patient-input device, such as external programmer 150 or a patient device such as the patient's phone and/or computing device. In the example of FIG. 2B, the processing of patient feedback information occurs in a device other than IMD 200B. Referring again to FIG. 2A, the patient feedback unit 216, discussed further below, receives information regarding the patient feedback data, such as information relating to sensed and/or received patient feedback associated with the efficacy of the electrical stimulation therapy, and controls the electrical stimulation circuitry 202 to deliver the electrical stimulation to the patient based on the received information, where the indications of the received information may be stored in a storage device. Processing circuitry 210A and/or 210B also controls stimulation generation circuitry 202 to generate and apply the stimulation signals to selected combinations of electrodes 232A, 232B. In some examples, stimulation generation circuitry 202 includes a switch circuit (instead of, or in addition to, switch circuitry 204) that may couple stimulation signals to selected conductors within leads 230, which, in turn, deliver the stimulation signals across selected electrodes 232A, 232B. Such a switch circuit may selectively couple stimulation energy to selected electrodes 232A, 232B and to selectively sense bioelectrical neural signals of a spinal cord of the patient with selected electrodes 232A, 232B. In other examples, however, stimulation generation circuitry 202 does not include a switch circuit and switch circuitry 204 does not interface between stimulation generation circuitry 202 and electrodes 232A, 232B. In these examples, stimulation generation circuitry 202 may include a plurality of pairs of current sources and current sinks, each connected to a respective electrode of electrodes 232A, 232B. In other words, in these examples, each of electrodes 232A, 232B is independently controlled via its own stimulation circuit (e.g., via a combination of a regulated current source and sink), as opposed to switching stimulation signals between different electrodes of electrodes 232A, 232B.

Storage device 212A and/or 212B may be configured to store information within IMD 200A and/or 200B, respectively, during operation. Storage device 212A and/or 212B may include a computer-readable storage medium or computer-readable storage device. In some examples, storage device 212A and/or 212B includes one or more of a short-term memory or a long-term memory. Storage device 212A and/or 212B may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, storage device 212A and/or 212B is used to store data indicative of instructions, e.g., for execution by processing circuitry 210A and/or 210B, respectively. As discussed above, storage device 212A and/or 212B is configured to store stimulation parameter settings 242.

Power source 224A and/or power source 224B may be configured to deliver operating power to the components of IMD 200A and/or 200B. Power source 224A and/or power source 224B may include a battery and a power generation circuit to produce the operating power. In some examples, the battery is rechargeable to allow extended operation. In some examples, recharging is accomplished through proximal inductive interaction between an external charger and an inductive charging coil within IMD 200A and/or 200B. Power source 224A and/or power source 224B may include any one or more of a plurality of different battery types, such as nickel cadmium batteries and lithium ion batteries. In some examples, power sources 224A and 224B may be primary cell devices, and in some examples power sources 224A and 224 may be different from each other, e.g., power source 224A may be a rechargeable battery and power source 224B may be a primary cell device.

In some examples, power source 224B may be configured to deliver operating power such that IMD 200A and/or IMD 200B may deliver one or more maintenance doses of stimulation over a period of time but may not be capable of delivering one or more higher-power consuming loading and/or rescue doses. In some examples, power source 224A may be configured to deliver operating power such that IMD 200A and/or IMD 200B may deliver one or more doses of either a loading dose or a maintenance dose. For example, a first IMD 200A and/or IMD 200B may include a power source 224A configured to deliver operating power such that the first IMD 200A and/or IMD 200B may deliver one or more loading doses over a first period of time, e.g., seconds, minutes, hours, days, weeks, months, etc. First IMD 200A and/or IMD200B, or programmer 300, may determine a maintenance dose based on patient feedback received by one or more sensors 160 or directly from the patient via a patient-input device. A second IMD 200A and/or IMD 200B may include a power source 224B configured to deliver a lesser amount of operating power and that is not capable of providing the power needed for the loading dose but is capable of providing the power needed for the maintenance dose. In some examples, the power source 224A may be switched with a smaller power source 224B in the first IMD 200A and/or IMD 200B rather than switching the entire IMD. In some examples, stimulation circuitry 202 switch circuitry 204, and/or processing circuitry 210A and/or processing circuitry 210B may limit the operating power that power source 224A may deliver such that the first IMD 200A and/or IMD 200B is no longer capable of providing the loading dose but may still be capable of delivering the maintenance dosing, e.g., effectively limiting power source 224A to be a power source 224B. In some examples, switching the first IMD 200A and/or IMD 200B, switching the power source 224A, and/or limiting the operating power output by power source 224A may increase a battery life of IMD 200A and/or IMD 200B.

In some examples, the second IMD 200A and/or IMD 200B using power source 224B may be configured to deliver a rescue dose of stimulation that consumes an amount of power greater than the maintenance dosing. For example, IMD 200A and/or IMD 200B may be configured to be connected to external power such that it is capable of delivering one or more rescue doses. In some examples, the rescue dose may be substantially the same as the loading dose. In some examples, stimulation circuitry 202 switch circuitry 204, and/or processing circuitry 210A and/or processing circuitry 210B may remove a limit on the operating power that power source 224A may deliver such that the second IMD 200A and/or IMD 200B is capable of providing the rescue dose.

In some examples as shown in FIG. 2A, the processing circuitry 210A of the IMD 200A directs delivery of electrical stimulation by the electrodes 232A, 232B of leads 230A, 230B, receives information relating to patient feedback from the patient feedback sensors or patient-input device, and generates output based on the received information. The patient feedback unit 216 may use patient feedback information to develop recommended electrical stimulation parameters or adjustments which are outputted to a user, where the user can use the indications or one or more recommended stimulation parameters to program the IMD 200A, e.g., by selecting or accepting the recommendations as stimulation parameter settings to be used by IMD 200A. For example, a particular cycling and/or a set of stimulation parameters are recommended to a user and presented to the user via the programmer. The user may accept the recommended cycling and/or one or more recommended stimulation parameters, and the programmer programs IMD 200A to implement and deliver stimulation with the selected electrode combination and/or stimulation parameters.

Processing circuitry 210A and/or 210B controls stimulation circuitry 202 to deliver stimulation energy with stimulation parameters specified by one or more stimulation parameter settings 242 stored on storage device 212A and/or 212B and, in the example of FIG. 2A, to collect patient feedback information pertaining to the stored stimulation parameter settings 242. Processing circuitry 210A and/or 210B collects this patient feedback information by receiving the information via telemetry from a remote patient feedback sensor and/or patient-input device at a remote site. Processing circuitry 210A may also control stimulation circuitry 202 to test different parameter settings and record corresponding patient feedback data for each selected combination, and test different parameter settings as they compare to sensed and/or patent-input patient feedback. For example, processing circuitry 210A directs stimulation circuitry 202 to deliver stimulation via a particular cycling and the patient feedback unit 216 collects the corresponding patient feedback data from telemetry circuitry 208. The patient feedback data 254 for this test may be stored in the storage device 212A. Processing circuitry 210A may adjust the previously tested cycling of the stimulation delivered via the electrode combination to a different cycling and collect the corresponding patient feedback data from the patient feedback sensor and/or patient-input device in response to stimulation with the adjusted cycling. The patient feedback data received for the stimulation at the changed stimulation parameter, in this example, cycling, would be saved in the storage device 212A and may be output to a user. The processing circuitry 210A may continue to shift the cycling by either increasing or decreasing the cycling frequency and/or cycling duty cycle, and recording the respective patient feedback data, which is stored on the storage device 212A and the information may be output to a user. While the example of cycling is provided, processing circuitry 210A may direct stimulation circuitry 202 to step through various incremental settings of other stimulation parameters, such as stimulation amplitude, pulse width, stimulation frequency, and record the respective patient feedback information for each stepped value. In one or more examples, processing circuitry 210A may direct stimulation circuitry to turn on for a certain period of time, and/or to turn off for a period of time, or to turn on at a certain time of day and record the respective patient feedback. Stimulation circuitry 202 may shift more than one stimulation parameter for each test and collect sensed patient feedback information for each of the multiple shifted stimulation parameters.

In some examples, the patient feedback unit 216 processes the patient feedback information to perform closed-loop control of the stimulation parameters based on the patient feedback information. The patient feedback unit 216 may store the patient feedback data 254 in storage device 212A. For example, patient feedback unit 216 may select or adjust one or more settings of parameter values, such as electrode combination, amplitude, pulse width or pulse rate, or cycling in response to patient feedback information. The patient feedback information may be collected when electrical stimulation is not delivered or upon delivery of electrical stimulation.

In some examples, the processing circuitry 210A and/or 210B of the IMD 200A and/or 200B, respectively, directs delivery of electrical stimulation of the electrodes 232A, 232B, and receives information relating to patient feedback from one or more patient feedback sensors 160, either directly (e.g., in the case of processing circuitry 210A) or via external controller (e.g., in the case of processing circuitry 210B), and controls the delivery of electrical stimulation of the electrodes 232A, 232B based on the received information in a closed loop setting. The patient feedback information may be received via the telemetry circuitry 208 either directly or indirectly from the patient feedback sensor 160 (FIG. 1). In an example, the IMD 200A and/or IMD 200B may receive the patient feedback information from an intermediate device other than the patient feedback sensor, such as external programmer 150.

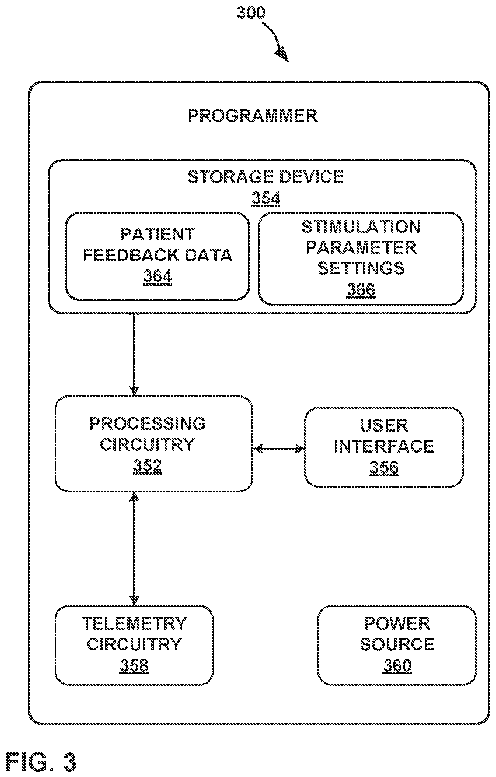

FIG. 3 is a block diagram illustrating an example configuration of components of an example external programmer 300. External programmer 300 may be an example of external programmer 150 of FIG. 1. Although external programmer 300 may generally be described as a hand-held device, such as a tablet computer or smartphone-like device, external programmer 300 may be a larger portable device, such as a laptop computer ,or a more stationary device, such as a desktop computer. In addition, in other examples, external programmer 300 may be included as part of an external charging device or include the functionality of an external charging device, e.g., to recharge a battery or batteries associated with IMD 200. As illustrated in FIG. 3, external programmer 300 may include processing circuitry 352, storage device 354, user interface 356, telemetry circuitry 358, and power source 360. In some examples, storage device 354 may store instructions that, when executed by processing circuitry 352, cause processing circuitry 352 and external programmer 300 to provide the functionality ascribed to external programmer 300 throughout this disclosure. Each of these components, circuitry, or modules, may include electrical circuitry that is configured to perform some, or all of the functionality described herein. For example, processing circuitry 352 may include processing circuitry configured to perform the processes discussed with respect to processing circuitry 352.

In general, external programmer 300 includes any suitable arrangement of hardware, alone or in combination with software and/or firmware, to perform the techniques attributed to external programmer 300, and processing circuitry 352, user interface 356, and telemetry circuitry 358 of external programmer 300. In various examples, processing circuitry 352, telemetry circuitry 358, or other circuitry of external programmer 300 may include one or more processors, such as one or more microprocessors, DSPs, ASICs, FPGAs, or any other equivalent integrated or discrete logic circuitry, as well as any combinations of such components. External programmer 300 also, in various examples, may include a storage device 354, such as RAM, ROM, PROM, EPROM, EEPROM, flash memory, a hard disk, a CD-ROM, including executable instructions for causing the one or more processors to perform the actions attributed to them. Moreover, although processing circuitry 352 and telemetry circuitry 358 are described as separate modules, in some examples, processing circuitry 352 and telemetry circuitry 358 are functionally integrated. In some examples, processing circuitry 352, telemetry circuitry 358 or other circuitry of external programmer 300 may correspond to individual hardware units, such as ASICs, DSPs, FPGAs, or other hardware units.

The processing circuitry 352 is configured to direct delivery of electrical stimulation, receive information relating patient feedback. In some examples, the processing circuitry 352 is configured to control the electrical stimulation circuitry to deliver the electrical stimulation based on the patient feedback information in a closed loop basis by directing the IMD to use particular stimulation parameters.

Storage device 354 (e.g., a storage device) may, in some examples, store instructions that, when executed by processing circuitry 352, cause processing circuitry 352 and external programmer 300 to provide the functionality ascribed to external programmer 300 throughout this disclosure. For example, storage device 354 may include instructions that cause processing circuitry 352 to obtain a parameter set from memory or receive user input and send a corresponding command to IMD 200, or instructions for any other functionality. In addition, storage device 354 may include a plurality of programs, where each program includes a parameter set that defines therapy stimulation or control stimulation. Storage device 354 may also store data received from a medical device (e.g., IMD 110) and/or a remote sensing device. For example, storage device 354 may store data recorded at a sensing module of the medical device, and storage device 354 may also store data from one or more sensors of the medical device. In an example, storage device 354 may store data recorded at a remote sensing device such as patient feedback from one or more sensors and/or patient-input devices.

User interface 356 may include a button or keypad, lights, a speaker for voice commands, a display, such as a liquid crystal (LCD), light-emitting diode (LED), or organic light-emitting diode (OLED). In some examples, the display includes a touch screen. User interface 356 may be configured to display any information related to the delivery of electrical stimulation including output, for example, based on the patient feedback information. User interface 356 may also receive user input (e.g., indication of when the patient perceives stimulation, or a pain score perceived by the patient upon delivery of stimulation) via user interface 356. The user input may be, for example, in the form of pressing a button on a keypad or selecting an icon from a touch screen. The input may request starting or stopping electrical stimulation, the input may request a new electrode combination or a change to an existing electrode combination, or the input may request some other change to the delivery of electrical stimulation, such as a change in stimulation cycling amplitude, pulse width or pulse rate.

Telemetry circuitry 358 may support wireless communication between the medical device and external programmer 300 under the control of processing circuitry 352. Telemetry circuitry 358 may also be configured to communicate with another computing device via wireless communication techniques, or direct communication through a wired connection. In some examples, telemetry circuitry 358 provides wireless communication via an RF or proximal inductive medium. In some examples, telemetry circuitry 358 includes an antenna, which may take on a variety of forms, such as an internal or external antenna.

Examples of local wireless communication techniques that may be employed to facilitate communication between external programmer 300 and IMD 110 include RF communication according to the 802.11 or Bluetooth ^{®} specification sets or other standard or proprietary telemetry protocols. In this manner, other external devices may be capable of communicating with external programmer 300 without needing to establish a secure wireless connection. As described herein, telemetry circuitry 358 may be configured to transmit a spatial electrode movement pattern or other stimulation parameters to IMD 110 for delivery of electrical stimulation therapy.

Power source 360 is configured to deliver operating power to the components of external programmer 300. Power source 360 may include a battery and a power generation circuit to produce the operating power. In some examples, the battery is rechargeable to allow extended operation. Recharging may be accomplished by electrically coupling power source 360 to a cradle or plug that is connected to an alternating current (AC) outlet. In addition, recharging may be accomplished through proximal inductive interaction between an external charger and an inductive charging coil within external programmer 300. In other examples, traditional batteries (e.g., nickel cadmium or lithium ion batteries) may be used. In addition, external programmer 300 may be directly coupled to an alternating current outlet to operate.

In some examples, the extemal programmer 300 or external control device directs delivery of electrical stimulation of an IMD, receives information relating to patient feedback, and generates output based on the received information, e.g., for evaluation of efficacy of stimulation parameters and/or to recommend or assist a user in programming stimulation parameters for delivery of electrical stimulation, or used as part of a closed loop control device to automatically adjust stimulation parameters using patient feedback information. In one or more examples, the control device generates output based on a first received information and a second received information via a user interface device.

Programmer 300 may be a patient programmer or a clinician programmer and receives patient feedback information such as patient feedback data 364. Programmer 300 receives patient feedback information and allows a user to interact with the processing circuitry 352 via user interface 356 in order to identify efficacious parameter settings, such as cycling and/or one or more other stimulation parameters using the patient feedback information. Programmer 300 further assists the user in programming a neurostimulation device by using the patient feedback information displayed on the user interface 356. In addition, programmer 300 may be used as part of a closed loop control device to automatically adjust stimulation parameters based at least on patient feedback information. In some examples, programmer 300 receives patient feedback information such as patient feedback data 364 from the patient feedback device and stores the patient feedback data in the storage device 354.

Programmer 300 may be used to determine efficacy of particular parameter settings of the IMD by testing parameter settings and recording patient feedback for each parameter setting. Information resulting from the testing may be presented to a user via the user interface 356. Programmer 300 may receive user input via the user interface device following generation of the output based on the first received information and the second received information, selecting one or more stimulation parameters for the delivery of the electrical stimulation. In one or more examples, the programmer 300 may generate a third set of stimulation parameters for delivery of the electrical stimulation based on the user input. In some examples, the programmer 300 compares the first information that was received relating to the first patient feedback with the second information relating to the second patient feedback, and automatically generates a third set of stimulation parameters for delivery of the electrical stimulation based on the comparison.

In an example, programmer 300 may be used to cause the IMD to automatically scan though a plurality of electrode combinations or parameter combinations. Processing circuitry 352 causes the IMD to automatically scan through each of a plurality of parameter combinations, including electrode combinations and parameter combinations. For each combination, the programmer 300 obtains and records the corresponding patient feedback.

Alternative to or in addition to the automatic scanning process, the user could manually advance scanning through electrode pairs and/or parameter combinations, for example with an arrow button on user interface 356. In some examples, as the user scans through the electrode pairs or parameter combinations to test and record patient feedback for each combination, the user may collect information such as a patient pain score indicating the degree of pain relief information from the combination, or a stimulation perception score indicating whether the patient perceives the stimulation, e.g., by verbal interaction with the patient or patient entry of information via a user input device, and enter the pain information into programmer 300 via user interface 356 of the programmer or the user input device.

Processing circuitry 352 controls stimulation circuitry 202 to deliver stimulation energy with stimulation parameters specified by one or more stimulation parameter settings 366 stored on storage device 354, and to collect patient feedback information pertaining to the stored stimulation parameter settings 366. Processing circuitry 352 may also control stimulation circuitry 202 to test different parameter settings and record corresponding patient feedback for each selected combination, and test different parameter settings as they compare to patient feedback. For example, processing circuitry 352 directs stimulation circuitry 202 to deliver stimulation with a particular dose and patient feedback is collected from telemetry circuitry 358. The patient feedback data 364 for this test may be stored in the storage device 354.

Processing circuitry 352 may be configured to change the previously tested dose to a different dose and collect the corresponding patient feedback. The patient feedback received for the stimulation at the changed stimulation parameter, for example cycling, would be saved in the storage device 354. The processing circuitry 352 may continue to change the dose by either increasing or decreasing the dose via any of the stimulation parameters, and record the respective patient feedback, which are stored on the storage device 354 and the information is output, e.g., via user interface 356. While the example of cycling is provided, processing circuitry 352 may direct stimulation circuitry to step through various incremental settings of other stimulation parameters, such as stimulation amplitude, stimulation pulse width, or stimulation frequency, and record the respective patient feedback information for each stepped value. Stimulation circuitry 202 may change and/or shift more than one stimulation parameter for each test and collect patient feedback information for the multiple shifted stimulation parameters.

In some examples, the processing circuitry 352 of programmer 300 directs delivery of electrical stimulation of the electrodes 232A, 232B, and receives information relating to patient feedback, and controls the delivery of electrical stimulation of the electrodes 232A, 232B based on the received patient feedback information in a closed loop setting. The patient feedback information may be received via the telemetry circuitry 358 either directly or indirectly from sensor 160 (FIG. 1) and/or a patient-input device.

The architecture of external programmer 300 illustrated in FIG. 3 is shown as an example. The techniques as set forth in this disclosure may be implemented in the example external programmer 300 of FIG. 3, as well as other types of systems not described specifically herein. Nothing in this disclosure should be construed so as to limit the techniques of this disclosure to the example architecture illustrated by FIG. 3.

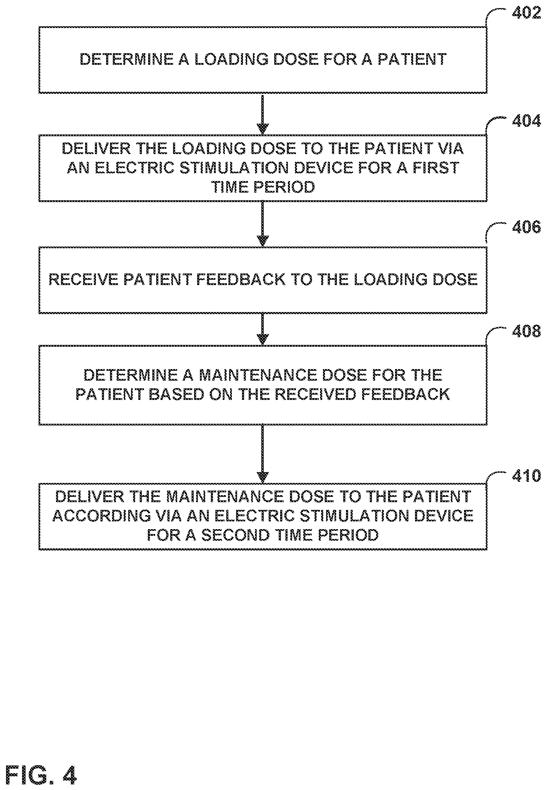

FIG. 4 is a flow diagram illustrating an example method of titrating a therapy, in accordance with one or more techniques of this disclosure. Although FIG. 4 is discussed using IMD 200A and/or IMD 200B of FIG. 2A and external programmer 300 of FIG. 3, it is to be understood that the methods discussed herein may include and/or utilize other systems and methods in other examples.

A physician or clinician, IMD 200A, or external programmer 300 may determine a loading dose for a patient (402). For example, a physician or clinician may select values for a number of programmable stimulation parameters in order to define the electrical stimulation therapy to be delivered by the IMD 200A to the patient and may input the parameters as stimulation parameter settings 242 and/or 366, e.g., via user interface 356 of external programmer 300. The selected values for the stimulation parameters may include a loading dose set of pulse parameters and a loading dose set of cycling parameters. In some examples, processing circuitry 210A, 210B, and/or 352 may determine and/or select the values for a number or programmable stimulation parameters, including the loading dose pulse and cycling parameters, according to executable instructions and, for example, based on information from sensors 222 in response to previous electric stimulation delivered to the patient.

IMD 200A may deliver one or more electric stimulation loading doses to the patient according to the loading dose stimulation parameters for a first time period (404). For example, processing circuitry 210A may control stimulation circuitry 202 to deliver stimulation energy via electrodes 232A, 232B with stimulation parameters specified by one or more stimulation parameter settings 242 stored on storage device 212A, such as the determined loading dose for a number of seconds, minutes, hours, days, weeks, months, or any other suitable time period.

IMD 200A may receive patient feedback representing a response of the patient to the one or more electric stimulation loading doses delivered according to the loading dose stimulation parameters (406). For example, processing circuitry 210A may control stimulation circuitry 202, telemetry circuitry, and/or sensors 222 to collect patient feedback information, e.g., patient feedback data 254, by receiving the information via telemetry from a remote patient feedback sensor and/or patient-input device at a remote site. Processing circuitry 210A may store received patient feedback data 254 in storage device 212A. On some examples, IMD 200A may receive patient feedback as one or more of physiological signals, patient input, patient posture data, or any other suitable patient feedback type, signal, input, and the like. For example, IMD 200A may receive one or more ECAP and/or ECAP signals, LFPs, a heart rate, a heart rate variability, a blood flow, a galvanic skin response, a network excitability, a signal and/or information related to a circadian rhythm, and the like. In some examples, IMD 200A may receive a pain response, a pain score, an area of pain, an amount of paresthesia, an area of paresthesia, a signal and/or information relating to voiding and/or a voiding rate (e.g., voids per day), and the like. In some examples, IMD 200A may receive patient posture and/or patient behavior data such as patient position, patient movement, patient movement history over a predetermined amount of time, a history of patent-selected stimulation parameters over a predetermined amount of time, and the like.

IMD 200A may determine, based on the patient feedback, a maintenance dose of the electric stimulation (408). In some examples, IMD 200A may determine, based on the feedback, that the patient does not need as large an amount of electric stimulation pulses (e.g., relating to the electrode combination, amplitude, pulse width and pulse frequency), and/or that the patient does not need electric stimulation doses as often (e.g., relating to electric stimulation cycling rate or frequency or period) or for as long for each dose (e.g., relating to electric stimulation on-time, off-time, or cycling duty cycle). For example, the patient may be responding well to the therapy and doesn't need as much electric stimulation and the electric stimulation dose may be reduced after the first period of time. IMD 200A may determine a maintenance dose of the electric stimulation with a reduce pulse (e.g., differing electrode combination, reduced amplitude, pulse width, and/or pulse frequency), a reduced cycling frequency and/or a reduced electric stimulation on-time (or an increased off-time) for each cycle. In other words, IMD 200A may determine a maintenance dose of the electric stimulation where an amount of on-time of each cycle, the cycling frequency, the pulse amplitude, the pulse width, and/or the pulse frequency is less than the amount of on-time of each cycle, the cycling frequency, the pulse amplitude, the pulse width, and/or the pulse frequency of the loading dose. In some examples, IMD 200A may determine a maintenance dose that consumes less power of IMD 200A than the loading dose. In some examples, IMD 200A may determine the maintenance dose as part of an electric stimulation titration tapering process, e.g., via decreasing the electric stimulation dose via one of reduced cycling and/or reducing other stimulation parameter settings 242, namely, the electrode combination, pulse amplitude, pulse width, and/or pulse frequency of the stimulation doses, over the first time period.

IMD 200A may deliver one or more electric stimulation maintenance doses to the patient according to the maintenance dose stimulation parameters for a second time period (410). For example, processing circuitry 210A may control stimulation circuitry 202 to deliver stimulation energy via electrodes 232A, 232B with stimulation parameters specified by one or more stimulation parameter settings 242 stored on storage device, such as the determined maintenance dose. In some examples, IMD 200A may deliver one or more electric stimulation maintenance doses that consume less power of IMD 200A than the loading dose.

In some examples, a different device and/or IMD, e.g., IMD 200B, may deliver one or more electric stimulation maintenance doses to the patient according to the maintenance dose stimulation parameters for the second time period. In other words, IMD 200A may be a first device configured to deliver the loading doses, and IMD 200B may be a second device configured to deliver the maintenance doses, and IMD 200B may not be configured to independently deliver the loading doses, e.g., power source 224B may not be capable of delivering the loading dose. In some examples, IMD 200B, e.g., the second device, may be configured to deliver the loading doses when connected to external power. In some examples, the second device, e.g., IMD 200B in this example, may be smaller, lighter, less costly, and use a different power source 224B than the first device, e.g., IMD 200A. In some examples, the second device (IMD 200B) may be implantable whereas the first device (IMD 200A) is not. In some examples, both the first and second devices may be implantable.

In some examples, a single device, e.g., IMD 200A and/or IMD 200B, may be configured to deliver loading doses when connected to external power during the first time period, and to deliver maintenance doses (without being connected to external power) during the second time period. For example, IMD 200B may not be configured to independently deliver the loading doses, e.g., power source 224B may not be capable of delivering the loading dose but may nevertheless deliver the loading device while connected to an external power source.

In some examples, IMD 200A and/or IMD 200B may receive patient feedback representing a response of the patient to the one or more maintenance doses, e.g., further patient feedback after titration via delivery of the one or more maintenance doses during and/or after the second time period. For example, the patient may start to do worse such that the patient may require more stimulation therapy. In some examples, processing circuitry 210A and/or 210B may control stimulation circuitry 202, telemetry circuitry, and/or sensors 222 to collect patient feedback information, e.g., patient feedback data 254, such as described above. IMD 200A may determine, based on the patient feedback, to deliver one or more rescue doses to the patient. For example, IMD 200A may determine to switch the stimulation therapy to higher dose, e.g., one that consumes more power. In some examples, IMD 200A may then deliver one or more electric stimulation rescue doses to the patient. In some examples, IMD 200A may be connected to external power in order to deliver the one or more rescue doses, and in some examples, the one or more rescue doses may be substantially similar to the one or more loading doses.

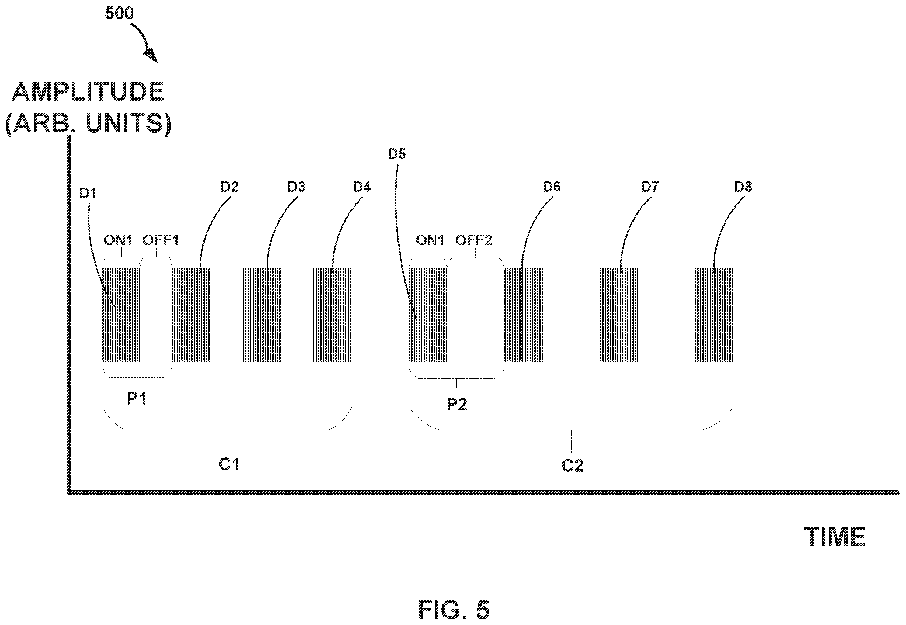

FIGS. 5-9 are a series of plots 500-900, respectively, illustrating examples of titrating electric stimulation doses, in accordance with one or more techniques of this disclosure. The series of plots illustrate examples of titrating electric stimulation doses via titrating stimulation cycle frequency, stimulation cycle duty cycle, both stimulation cycle frequency and duty cycle, and continuous titration tapering.

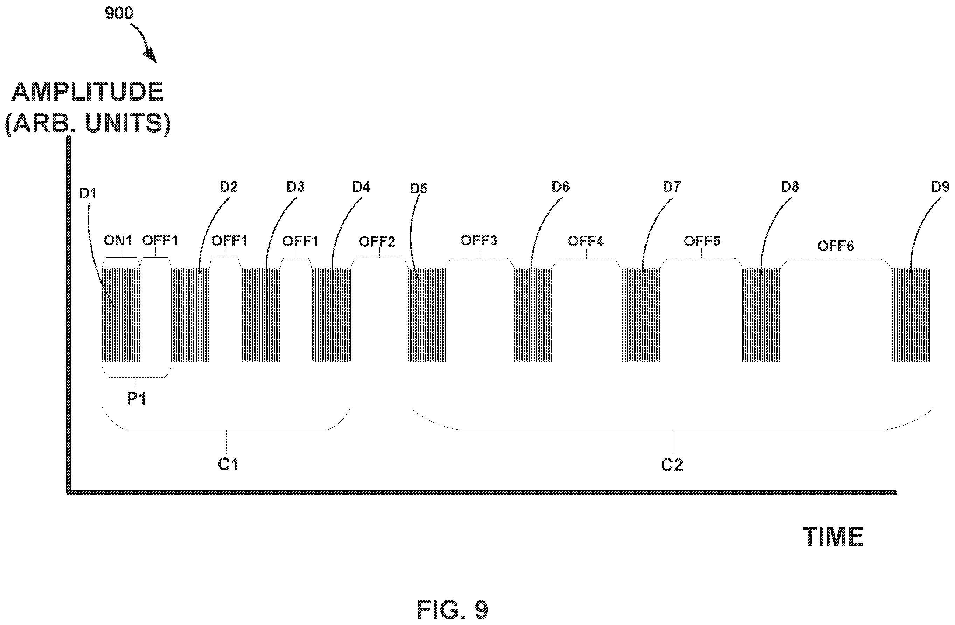

In the examples shown in each of FIGS. 5-9, each of doses D1-D8 (and D9 in FIG. 9) are substantially similar, e.g., each having a substantially similar electrode combination, amplitude, pulse frequency and pulse width. In some examples, each of doses D1-D9 may be different from each other, e.g., having different electrode combinations, amplitudes, pulse frequencies and pulse widths, or some may be substantially the same and some may be different. Although first cycling C1 and second cycling C2 each have four doses in FIGS. 5-8, it is to be understood that first cycling C1 and second cycling C2 may include any number of doses.

FIG. 5 is a plot 500 of an example of titrating electric stimulation doses, in accordance with one or more techniques of this disclosure. Plot 500 illustrates titrating electric stimulation doses via increasing the off-time between doses, resulting in reducing the frequency of the cycling as well. In the example shown, first cycling C1 includes substantially similar doses D1-D4 each having an on-time ON1 and an off-time OFF1. The frequency of first cycling C1 is the reciprocal of the cycle period P1 (e.g., 1/P1), and the duty cycle is the ratio ON1/OFF1. Second cycling C2 includes substantially similar doses D5-D8 each having an on-time ON1 and an off-time OFF2. The frequency of second cycling C2 is the reciprocal of the cycle period P2 (e.g., 1/P2), and the duty cycle is the ratio ON1/OFF2. In the example shown, the electric stimulation is titrated down via increasing the off-time, e.g., OFF2 is greater than OFF1. As a result, an IMD delivering electric stimulation doses according to cycling C2 consumes less power than delivering electric stimulation doses according to cycling C1, and the amount of on-time over a period of time is reduced, e.g., the amount of on-time for second cycling C2 is less than for first cycling C1 over a period of time, and the cycling frequency of second cycling C2 is less than first cycling C1. Additionally, the duty cycle of the second cycling is reduced by virtue of the increase in off-time, e.g., OFF2 > OFF1.

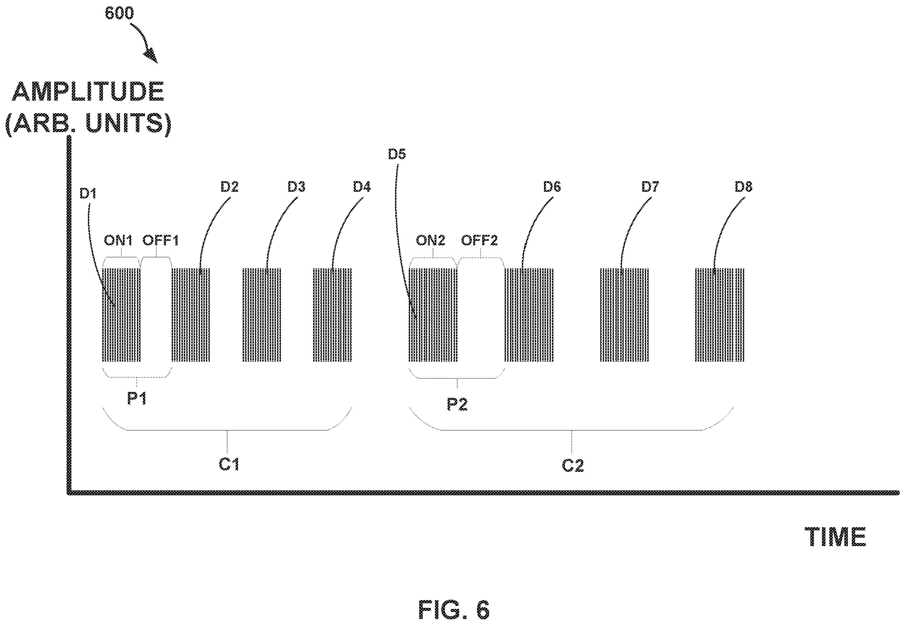

FIG. 6 is a plot 600 of an example of titrating electric stimulation doses, in accordance with one or more techniques of this disclosure. Plot 600 illustrates titrating electric stimulation doses via reducing the dosing frequency, however, the on-time of doses D5-D8 are increased to keep the duty cycle of second cycling C2 the same as C1. For example, the ratio of ON1/OFF1 may be substantially the same as ON2/OFF2 in the example of FIG. 6. Although titrating electric stimulation according to the example of FIG. 6 may not necessarily reduce the stimulation and/or power consumed by an IMD delivering the stimulation over time, reducing the frequency of the stimulation may be a useful intermediate step that may allow a patient to acclimate to a reduction in the amount of stimulation therapy via further titration in the future and/or to reduce the likelihood of developing a tolerance to the therapy by reducing its frequency but not the time-average of dosing received by the patient.

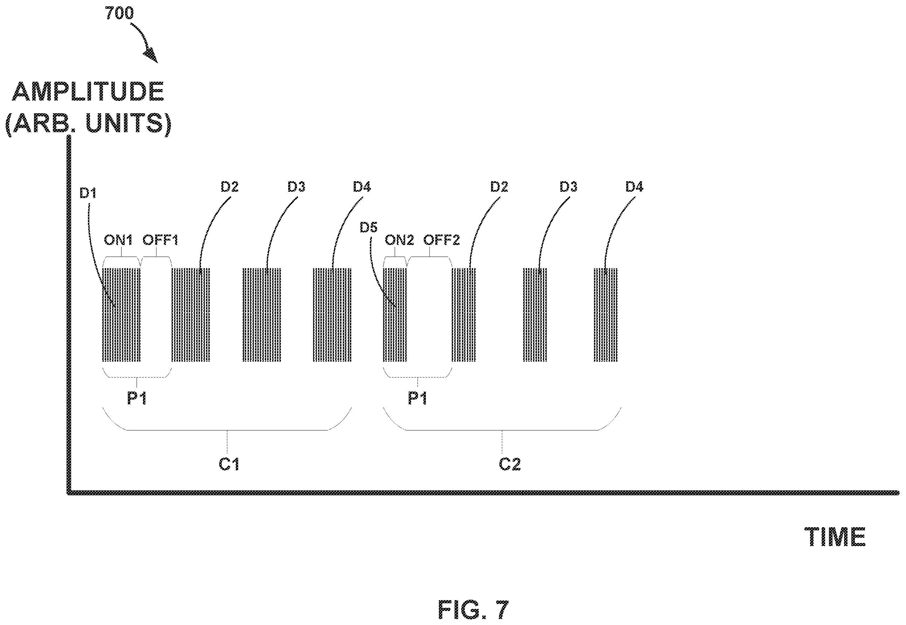

FIG. 7 is a plot 700 of an example of titrating electric stimulation doses, in accordance with one or more techniques of this disclosure. Plot 700 illustrates titrating electric stimulation doses via reducing the on-time of the doses. In the example shown, the frequency of each of first cycling C1 and second cycling C2 are kept the same by virtue of increasing the off-time by the same amount as the decrease in on-time, e.g., P1 = P2, however the on-time of second cycling C2 is reduced relative to first cycling C1, e.g., ON2 < ON1. Consequently, the duty cycle of the second cycling is also reduced, and an IMD delivering electric stimulation doses according to cycling C2 consumes less power than delivering electric stimulation doses according to cycling C1. Additionally, the amount of on-time, for each cycle as well as over a period of time, is reduced, e.g., the amount of on-time for second cycling C2 is less than for first cycling C1.

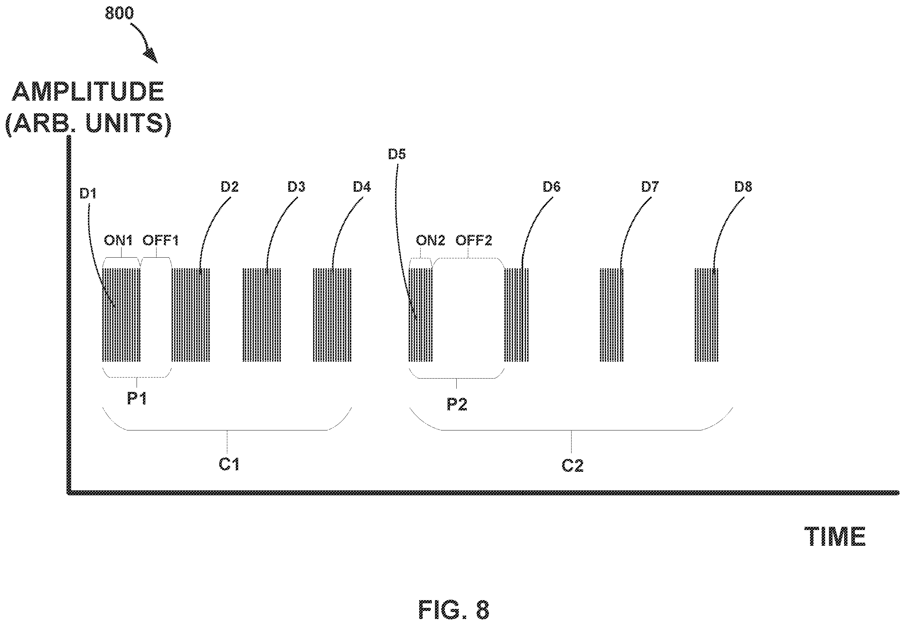

FIG. 8 is a plot 800 of an example of titrating electric stimulation doses, in accordance with one or more techniques of this disclosure. Plot 800 illustrates titrating electric stimulation doses via both increasing the off-time and reducing the on-time of the doses. Namely, ON2 < ON1, OFF2 > OFF1, and P2 > P1 (e.g., the frequency of the second cycling is reduced relative to the first cycling via an increase in the cycling period). As such, the duty cycle of the second cycling is reduced relative to the first cycling, and an IMD delivering electric stimulation doses according to cycling C2 consumes less power than delivering electric stimulation doses according to cycling C1.

FIG. 9 is a plot 900 of an example of titrating electric stimulation doses, in accordance with one or more techniques of this disclosure. Plot 900 illustrates titrating electric stimulation doses via a continuous taper of the electric stimulation doses by continuously increasing the off-time between the doses, e.g., OFF1 < OFF2 < OFF3 < OFF4 < OFF5 < OFF6. In the example shown, the first four does D1-D4 are delivered according to first cycling C1 with an on-time ON1, an off-time OFF1, a cycling frequency 1/P1, and a cycling duty cycle ON1/OFF1. In the example shown, the second cycling C2 does not have a constant period or off-time, but rather both increase between each of doses D5-D9. In the example shown, the on-times ON1 of all doses D1-D9 are substantially the same, however, it need not be so, and in some examples the on-time of any of doses D5-D9 may be increased and or decreased relative to ON1. In the example shown, the amount of electric stimulation delivered over the time period of second cycling C2 continuously decreases via the continuous increase in off-time and a continuous decrease in cycling frequency. In some examples, second cycling C2 may continuously decrease the on-time. In the example shown, an IMD delivering electric stimulation doses according to cycling C2 consumes less power than delivering electric stimulation doses according to cycling C1. The techniques described in this disclosure may be implemented, at least in part, in hardware, software, firmware or any combination thereof. For example, various aspects of the described techniques may be implemented within processing circuitry, which may include one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also form one or more processors or processing circuitry configured to perform one or more of the techniques of this disclosure.

Such hardware, software, and firmware may be implemented, and various operation may be performed within same device, within separate devices, and/or on a coordinated basis within, among or across several devices, to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components. Processing circuitry described in this disclosure, including a processor or multiple processors, may be implemented, in various examples, as fixed-function circuits, programmable circuits, or a combination thereof. Fixed-function circuits refer to circuits that provide particular functionality with preset operations. Programmable circuits refer to circuits that can be programmed to perform various tasks and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive stimulation parameters or output stimulation parameters), but the types of operations that the fixed-function circuits perform are generally immutable. In some examples, one or more of the units may be distinct circuit blocks (fixed-function or programmable), and in some examples, one or more of the units may be integrated circuits.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A system comprising:
electrodes configured to deliver the electrical stimulation to a patient; and
a device comprising processing circuitry (210A, 210B) configured to:
determine, for a patient, a loading dose of electric stimulation;
cause electrical first stimulation circuitry of a first stimulator device to deliver, during a first time period, the loading dose to the patient;
receive patient feedback representing a response of the patient to the loading dose;
determine, based on patient feedback, a maintenance dose of electrical stimulation; and
cause second electrical stimulation circuitry of a second stimulator device that is implantable in the patient to deliver, and during a second time period that is after the first time period, the maintenance dose of electrical stimulation, wherein delivering the maintenance dose of electrical stimulation consumes less power than delivering the loading dose of electrical stimulation.

2. The system of claim 1, wherein the second stimulator device is not independently capable of delivering the loading dose.

3. The system of claim 2, wherein causing the electrical stimulation circuitry to deliver the loading dose comprises causing the electrical stimulation circuitry to deliver the loading dose by the second stimulator device when the second stimulator device is connected to an external power source.

4. The system of any one of the preceding claims, wherein the first stimulator device is configured to deliver the loading dose via at least one electrode included with at least one lead, wherein the second stimulator device is configured to deliver the maintenance dose via the at least one electrode included with the at least one lead.

5. The system of any one of the preceding claims, wherein the processing circuitry (210A, 210B) is further configured to:
receive patient feedback representing a response of the patient to the maintenance dose;
based on the patient feedback, cause the second electrical stimulation circuitry to deliver a rescue dose of electrical stimulation during a third time period that is after the second time period and while the second stimulator device is connected to an external power source, wherein delivering the rescue dose of electrical stimulation consumes more power than delivering the maintenance dose of electrical stimulation.

6. The system of any one of claims 1-5, wherein the patient feedback comprises one or more of: an evoked compound action potential, a local field potential, a heart rate, a heart rate variability, a blood flow, a galvanic skin response, a network excitability, a pain response, pain score, an area of pain, an amount of paresthesia, an area of paresthesia, a voiding rate, a patient position, a patient movement, a patient movement history over a predetermined amount of time, and a history of patent-selected stimulation parameters over a predetermined amount of time.

7. The system of any of claims 1-6, wherein at least one of:
an amplitude of the maintenance dose is less than an amplitude of the loading dose,
a pulse width of the maintenance dose is less than a pulse width of the loading dose,
a pulse frequency of the maintenance dose is less than a pulse frequency of the loading dose,
a cycling frequency of the maintenance dose is less than a cycling frequency of the loading dose,
an amount of off-time of the maintenance dose is greater than an amount of off-time of the loading dose, or
an amount of on-time of the maintenance dose is less than an amount of on-time of the loading dose.

8. The system of any one of claims 1-7, wherein causing the electrical stimulation circuitry to deliver the loading dose comprises causing the electrical stimulation circuitry to deliver the loading dose according to a first cycling, wherein causing the electrical stimulation circuitry to deliver the maintenance dosing comprises causing the electrical stimulation circuitry to deliver the maintenance doses according to a second cycling, wherein a cycling frequency of the second cycling is less than a cycling frequency of the first cycling.

9. The system of claim 8, wherein an amount of on-time of each cycle of the second cycling is less than an amount of on-time of each cycle of the first cycling.

10. The system of claims 8 or 9, wherein an amount of off-time of each cycle of the second cycling is more than an amount of off-time of each cycle of the first cycling.

11. A computer readable medium comprising instructions that when executed by processing circuitry of a medical system comprising a first stimulator device and a second stimulator device that is implantable in the patient cause the processing circuitry (210A, 210B) to:
determine, for a patient, a loading dose of electric stimulation;
cause first electrical stimulation circuitry of the first stimulator device to deliver, during a first time period, the loading dose to the patient;
receive patient feedback representing a response of the patient to the loading dose;
determine, based on patient feedback, a maintenance dose of electrical stimulation;
and
cause second electrical stimulation circuitry of the second stimulator device that is implantable in the patient to deliver, and during a second time period that is after the first time period, the maintenance dose of electrical stimulation, wherein delivering the maintenance dose of electrical stimulation consumes less power than delivering the loading dose of electrical stimulation.

12. The computer readable medium of claim 11, wherein the second stimulator device is not independently capable of delivering the loading dose.

13. The computer readable medium of claim 12, wherein causing the electrical stimulation circuitry to deliver the loading dose comprises causing the electrical stimulation circuitry to deliver the loading dose by the second stimulator device when the second stimulator device is connected to an external power source.

14. The computer readable medium of any one of claims 11-13, wherein the patient feedback comprises one or more of: an evoked compound action potential, a local field potential, a heart rate, a heart rate variability, a blood flow, a galvanic skin response, a network excitability, a pain response, pain score, an area of pain, an amount of paresthesia, an area of paresthesia, a voiding rate, a patient position, a patient movement, a patient movement history over a predetermined amount of time, and a history of patent-selected stimulation parameters over a predetermined amount of time.

15. The computer readable medium of any one of claims 11-14, wherein at least one of:
an amplitude of the maintenance dose is less than an amplitude of the loading dose,
a pulse width of the maintenance dose is less than a pulse width of the loading dose,
a pulse frequency of the maintenance dose is less than a pulse frequency of the loading dose,
a cycling frequency of the maintenance dose is less than a cycling frequency of the loading dose,
an amount of off-time of the maintenance dose is greater than an amount of off-time of the loading dose, or
an amount of on-time of the maintenance dose is less than an amount of on-time of the loading dose.

## Patentansprüche

1. System, umfassend:
Elektroden, die konfiguriert sind, um die elektrische Stimulation an einen Patienten abzugeben; und
eine Vorrichtung, umfassend eine Verarbeitungsschaltung (210A, 210B), die konfiguriert ist zum:
Bestimmen, für einen Patienten, einer Beladungsdosis der elektrischen Stimulation;
Veranlassen einer ersten Schaltung der elektrischen Stimulation einer ersten Stimulatorvorrichtung, während einer ersten Zeitspanne, die Beladungsdosis an den Patienten abzugeben;
Empfangen einer Patientenrückmeldung, die eine Reaktion des Patienten auf die Beladungsdosis darstellt;
Bestimmen, basierend auf Patientenrückmeldungen, einer Erhaltungsdosis der elektrischen Stimulation; und
Veranlassen einer zweiten Schaltung der elektrischen Stimulation einer zweiten Stimulatorvorrichtung, die in den Patienten implantierbar ist, und während einer zweiten Zeitspanne, die nach der ersten Zeitspanne liegt, die Erhaltungsdosis der elektrischen Stimulation abzugeben, wobei das Abgeben der Erhaltungsdosis der elektrischen Stimulation weniger Leistung als das Abgeben der Beladungsdosis der elektrischen Stimulation verbraucht.

2. System nach Anspruch 1, wobei die zweite Stimulatorvorrichtung nicht unabhängig in der Lage ist, die Beladungsdosis abzugeben.

3. System nach Anspruch 2, wobei das Veranlassen der Schaltung der elektrischen Stimulation, die Beladungsdosis abzugeben, das Veranlassen der Schaltung der elektrischen Stimulation, die Beladungsdosis durch die zweite Stimulatorvorrichtung abzugeben, wenn die zweite Stimulatorvorrichtung mit einer externen Energiequelle verbunden ist, umfasst.

4. System nach einem der vorstehenden Ansprüche, wobei die erste Stimulatorvorrichtung konfiguriert ist, um die Beladungsdosis über mindestens eine Elektrode abzugeben, die in mindestens einer Lead eingeschlossen ist, wobei die zweite Stimulatorvorrichtung konfiguriert ist, um die Erhaltungsdosis über die mindestens eine Elektrode abzugeben, die in der mindestens einen Lead eingeschlossen ist.

5. System nach einem der vorstehenden Ansprüche, wobei die Verarbeitungsschaltung (210A, 210B) ferner konfiguriert ist zum:
Empfangen der Patientenrückmeldung, die eine Reaktion des Patienten auf die Erhaltungsdosis darstellt;
basierend auf der Patientenrückmeldung, Veranlassen der zweiten Schaltung der elektrischen Stimulation, während einer dritten Zeitspanne, die nach der zweiten Zeitspanne liegt und während die zweite Stimulatorvorrichtung mit einer externen Energiequelle verbunden ist, eine Rettungsdosis an elektrischer Stimulation abzugeben, wobei das Abgeben der Rettungsdosis an elektrischer Stimulation mehr Leistung als das Abgeben der Erhaltungsdosis an elektrischer Stimulation verbraucht.

6. System nach einem der Ansprüche 1 bis 5, wobei die Patientenrückmeldung eines oder mehrere umfasst von: einem evozierten Summenaktionspotenzial, einem lokalen Feldpotenzial, einer Herzfrequenz, einer Herzfrequenzvariabilität, einem Blutfluss, einer galvanischen Hautreaktion, einer Netzwerkerregbarkeit, einer Schmerzreaktion, einem Schmerzwert, einem Schmerzbereich, einem Ausmaß an Parästhesie, einem Parästhesiebereich, einer Miktionsrate, einer Patientenposition, einer Patientenbewegung, einem Verlauf der Patientenbewegung über eine vorbestimmte Zeitdauer und einem Verlauf von patientenausgewählten Stimulationsparametern über eine vorbestimmte Zeitdauer.

7. System nach Anspruch 1 bis 6, wobei mindestens eines von:
einer Amplitude der Erhaltungsdosis kleiner als eine Amplitude der Beladungsdosis ist,
einer Impulsbreite der Erhaltungsdosis kleiner als eine Impulsbreite der Beladungsdosis ist,
einer Pulsfrequenz der Erhaltungsdosis kleiner als eine Pulsfrequenz der Beladungsdosis ist,
einer Durchlauffrequenz der Erhaltungsdosis kleiner als eine Durchlauffrequenz der Beladungsdosis ist,
einer Dauer an Ausschaltzeit der Erhaltungsdosis größer als eine Dauer an Ausschaltzeit der Beladungsdosis ist, oder
einer Dauer der Einschaltzeit der Erhaltungsdosis kleiner als eine Dauer der Einschaltzeit der Beladungsdosis ist.

8. System nach einem der Ansprüche 1 bis 7, wobei das Veranlassen der Schaltung der elektrischen Stimulation, die Beladungsdosis abzugeben, das Veranlassen der Schaltung der elektrischen Stimulation, die Beladungsdosis gemäß einem ersten Durchlauf abzugeben, umfasst, wobei das Veranlassen der Schaltung der elektrischen Stimulation, die Erhaltungsdosierung abzugeben, das Veranlassen der Schaltung der elektrischen Stimulation, die Erhaltungsdosen gemäß einem zweiten Durchlaufen abzugeben, umfasst, wobei eine Durchlauffrequenz des zweiten Durchlaufens kleiner als eine Durchlauffrequenz des ersten Durchlaufens ist.

9. System nach Anspruch 8, wobei eine Dauer an Einschaltzeit jedes Durchlaufs des zweiten Durchlaufens kleiner als eine Dauer an Einschaltzeit jedes Durchlaufs des ersten Durchlaufens ist.

10. System nach Anspruch 8 oder 9, wobei eine Dauer der Ausschaltzeit jedes Durchlaufs des zweiten Durchlaufens größer als eine Dauer der Ausschaltzeit jedes Durchlaufs des ersten Durchlaufens ist.

11. Computerlesbares Medium, umfassend Anweisungen, die wenn sie durch eine Verarbeitungsschaltung eines medizinischen Systems ausgeführt werden, umfassend eine erste Stimulatorvorrichtung und eine zweite Stimulatorvorrichtung, die in den Patienten implantierbar ist, die Verarbeitungsschaltung (210A, 210B) veranlassen zum:
Bestimmen, für einen Patienten, einer Beladungsdosis der elektrischen Stimulation;
Veranlassen der ersten Schaltung der elektrischen Stimulation der ersten Stimulatorvorrichtung, während einer ersten Zeitspanne, die Beladungsdosis an den Patienten abzugeben;
Empfangen der Patientenrückmeldung, die eine Reaktion des Patienten auf die Beladungsdosis darstellt;
Bestimmen, basierend auf Patientenrückmeldungen, einer Erhaltungsdosis der elektrischen Stimulation; und
Veranlassen der zweiten Schaltung der elektrischen Stimulation der zweiten Stimulatorvorrichtung, die in den Patienten implantierbar ist, und während einer zweiten Zeitspanne, die nach der ersten Zeitspanne liegt, die Erhaltungsdosis der elektrischen Stimulation abzugeben, wobei das Abgeben der Erhaltungsdosis der elektrischen Stimulation weniger Leistung als das Abgeben der Beladungsdosis der elektrischen Stimulation verbraucht.

12. Computerlesbares Medium nach Anspruch 11, wobei die zweite Stimulatorvorrichtung nicht unabhängig in der Lage ist, die Beladungsdosis abzugeben.

13. Computerlesbares Medium nach Anspruch 12, wobei das Veranlassen der Schaltung der elektrischen Stimulation, die Beladungsdosis abzugeben, das Veranlassen der Schaltung der elektrischen Stimulation, die Beladungsdosis durch die zweite Stimulatorvorrichtung abzugeben, wenn die zweite Stimulatorvorrichtung mit einer externen Energiequelle verbunden ist, umfasst.

14. Computerlesbares Medium nach einem der Ansprüche 11 bis 13, wobei die Patientenrückmeldung eines oder mehrere umfasst von: einem evozierten Summenaktionspotenzial, einem lokalen Feldpotenzial, einer Herzfrequenz, einer Herzfrequenzvariabilität, einem Blutfluss, einer galvanischen Hautreaktion, einer Netzwerkerregbarkeit, einer Schmerzreaktion, einem Schmerzwert, einem **Schmerzbereich, einem Ausmaß an Parästhesie, einem Parästhesiebereich, einer** Miktionsrate, einer Patientenposition, einer Patientenbewegung, einem Verlauf der Patientenbewegung über eine vorbestimmte Zeitspanne und einem Verlauf von patientenausgewählten Stimulationsparametern über eine vorbestimmte Zeitspanne.

15. Computerlesbares Medium nach einem der Ansprüche 11 bis 14, wobei mindestens eines von:
einer Amplitude der Erhaltungsdosis kleiner als eine Amplitude der Beladungsdosis ist,
einer Impulsbreite der Erhaltungsdosis kleiner als eine Impulsbreite der Beladungsdosis ist,
einer Pulsfrequenz der Erhaltungsdosis kleiner als eine Pulsfrequenz der Beladungsdosis ist,
einer Durchlauffrequenz der Erhaltungsdosis kleiner als eine Durchlauffrequenz der Beladungsdosis ist,
einer Dauer an Ausschaltzeit der Erhaltungsdosis größer als eine Dauer an Ausschaltzeit der Beladungsdosis ist, oder
einer Dauer der Einschaltzeit der Erhaltungsdosis kleiner als eine Dauer der Einschaltzeit der Beladungsdosis ist.

## Revendications

1. Système comprenant :
des électrodes configurées pour administrer la stimulation électrique à un patient ; et
un dispositif comprenant une circuiterie de traitement (210A, 210B) configurée pour :
déterminer, pour un patient, une dose de stimulation électrique de charge ;
amener une première circuiterie de stimulation électrique d'un premier dispositif de stimulation à administrer, pendant un premier laps de temps, la dose de charge au patient ;
recevoir une rétroaction du patient représentant une réponse du patient à la dose de charge ;
déterminer, en fonction de la rétroaction du patient, une dose de stimulation électrique d'entretien ; et
amener une seconde circuiterie de stimulation électrique d'un second dispositif de stimulation qui est implantable dans le patient à administrer, et pendant un deuxième laps de temps qui est après le premier laps de temps, la dose de stimulation électrique d'entretien, dans lequel l'administration de la dose de stimulation électrique d'entretien consomme moins d'énergie que l'administration de la dose de stimulation électrique de charge.

2. Système selon la revendication 1, dans lequel le second dispositif de stimulation n'est pas capable indépendamment d'administrer la dose de charge.

3. Système selon la revendication 2, dans lequel le fait d'amener la circuiterie de stimulation électrique à administrer la dose de charge comprend le fait d'amener la circuiterie de stimulation électrique à administrer la dose de charge par le second dispositif de stimulation lorsque le second dispositif de stimulation est connecté à une source d'alimentation externe.

4. Système selon l'une quelconque des revendications précédentes, dans lequel le premier dispositif de stimulation est configuré pour administrer la dose de charge par l'intermédiaire d'au moins une électrode associée à au moins une sonde, dans lequel le second dispositif de stimulation est configuré pour administrer la dose d'entretien par l'intermédiaire de l'au moins une électrode associée à l'au moins une sonde.

5. Système selon l'une quelconque des revendications précédentes, dans lequel la circuiterie de traitement (210A, 210B) est en outre configurée pour :
recevoir une rétroaction du patient représentant une réponse du patient à la dose d'entretien ;
en fonction de la rétroaction du patient, amener la seconde circuiterie de stimulation électrique à administrer une dose de stimulation électrique de secours pendant un troisième laps de temps qui est après le deuxième laps de temps et alors que le second dispositif de stimulation est connecté à une source d'alimentation externe, dans lequel l'administration de la dose de stimulation électrique de secours consomme plus d'énergie que l'administration de la dose de stimulation électrique d'entretien.

6. Système selon l'une quelconque des revendications 1-5, dans lequel la rétroaction du patient comprend un ou plusieurs parmi : un potentiel d'action composé évoqué, un potentiel de champ local, une fréquence cardiaque, une variabilité de la fréquence cardiaque, un flux sanguin, une réponse cutanée galvanique, une excitabilité de réseau, une réponse à la douleur, un score de douleur, une zone de douleur, une quantité de paresthésie, une zone de paresthésie, un taux de miction, une position du patient, un mouvement du patient, un historique des mouvements du patient sur une durée prédéterminée, et un historique des paramètres de stimulation sélectionnés par le brevet sur une durée prédéterminée.

7. Système selon l'une quelconque des revendications 1-6, dans lequel au moins l'une parmi :
une amplitude de la dose d'entretien est inférieure à une amplitude de la dose de charge,
une largeur d'impulsion de la dose d'entretien est inférieure à une largeur d'impulsion de la dose de charge,
une fréquence d'impulsion de la dose d'entretien est inférieure à une fréquence d'impulsion de la dose de charge,
une fréquence de cycle de la dose d'entretien est inférieure à une fréquence de cycle de la dose de charge,
une quantité de temps d'arrêt de la dose d'entretien est supérieure à une quantité de temps d'arrêt de la dose de charge, ou
une quantité de temps d'activation de la dose d'entretien est inférieure à une quantité de temps d'activation de la dose de charge.

8. Système selon l'une quelconque des revendications 1-7, dans lequel le fait d'amener la circuiterie de stimulation électrique à administrer la dose de charge comprend le fait d'amener la circuiterie de stimulation électrique à administrer la dose de charge selon un premier cycle, dans lequel le fait d'amener la circuiterie de stimulation électrique à administrer le dose d'entretien comprend le fait d'amener la circuiterie de stimulation électrique à administrer les doses d'entretien selon un second cycle, dans lequel une fréquence de cycle du second cycle est inférieure à une fréquence de cycle du premier cycle.

9. Système selon la revendication 8, dans lequel une quantité de temps d'activation de chaque cycle du second cycle est inférieure à une quantité temps d'activation de chaque cycle du premier cycle.

10. Système selon les revendications 8 ou 9, dans lequel une quantité de temps d'arrêt de chaque cycle du second cycle est supérieure à une quantité de temps d'arrêt de chaque cycle du premier cycle.

11. Support lisible par ordinateur comprenant des instructions qui, lorsqu'elles sont exécutées par une circuiterie de traitement d'un système médical comprenant un premier dispositif de stimulation et un second dispositif de stimulation qui est implantable dans le patient, amènent la circuiterie de traitement (210A, 210B) à :
déterminer, pour un patient, une dose de stimulation électrique de charge ;
amener la première circuiterie de stimulation électrique du premier dispositif de stimulation à administrer, pendant un premier laps de temps, la dose de charge au patient ;
recevoir une rétroaction du patient représentant une réponse du patient à la dose de charge ;
déterminer, en fonction de la rétroaction du patient, une dose de stimulation électrique d'entretien ; et
amener une seconde circuiterie de stimulation électrique du second dispositif de stimulation qui est implantable dans le patient à administrer, et pendant un deuxième laps de temps qui est après le premier laps de temps, la dose de stimulation électrique d'entretien, dans lequel l'administration de la dose de stimulation électrique d'entretien consomme moins d'énergie que l'administration de la dose de stimulation électrique de charge.

12. Support lisible par ordinateur selon la revendication 11, dans lequel le second dispositif de stimulation n'est pas capable indépendamment d'administrer la dose de charge.

13. Support lisible par ordinateur selon la revendication 12, dans lequel le fait d'amener la circuiterie de stimulation électrique à administrer la dose de charge comprend le fait d'amener la circuiterie de stimulation électrique à administrer la dose de charge par le second dispositif de stimulation lorsque le second dispositif de stimulation est connecté à une source d'alimentation externe.

14. Support lisible par ordinateur selon l'une quelconque des revendications 11 à 13, dans lequel la rétroaction du patient comprend un ou plusieurs parmi : un potentiel d'action composé évoqué, un potentiel de champ local, une fréquence cardiaque, une variabilité de la fréquence cardiaque, un flux sanguin, une réponse cutanée galvanique, une excitabilité de réseau, une réponse à la douleur, un score de douleur, une zone de douleur, une quantité de paresthésie, une zone de paresthésie, un taux de miction, une position du patient, un mouvement du patient, un historique de mouvements du patient sur une durée prédéterminée, et un historique des paramètres de stimulation sélectionnés par le brevet sur une durée prédéterminée.

15. Support lisible par ordinateur selon l'une quelconque des revendications 11-14, , dans lequel au moins l'une parmi :
une amplitude de la dose d'entretien est inférieure à une amplitude de la dose de charge,
une largeur d'impulsion de la dose d'entretien est inférieure à une largeur d'impulsion de la dose de charge,
une fréquence d'impulsion de la dose d'entretien est inférieure à une fréquence d'impulsion de la dose de charge,
une fréquence de cycle de la dose d'entretien est inférieure à une fréquence de cycle de la dose de charge,
une quantité de temps d'arrêt de la dose d'entretien est supérieure à une quantité de temps d'arrêt de la dose de charge, ou
une quantité de temps d'activation de la dose d'entretien est inférieure à une quantité de temps d'activation de la dose de charge.
